# FASCICULE DE BREVET EUROPEEN

(11) **EP 3 237 490 B1**
(45) Date de publication et mention de la délivrance du brevet: **24.10.2018**
(21) Numéro de dépôt: 15828653.4
(22) Date de dépôt: 22.12.2015
(51) Int. Cl.: C08G 65/40, C08G 75/23

(54) **NOUVEAUX POLYMERES CONTENANT DES FONCTIONS SULFONATES METALLIQUES, LEURS PROCEDES DE PREPARATION ET LEURS UTILISATIONS COMME ANTIBACTERIENS, FONGICIDES, ANTIMICROBIENS**
NEUE METALLSULFONATENTHALTENDES POLYMERE, VERFAHREN ZU DEREN HERSTELLUNG UND DEREN VERWENDUNG ALS ANTIMIKROBIELLE, FUNGIZIDE, ANTIBAKTERIELLE MITTELN
NOVEL POLYMERS CONTAINING METALIC SULFONATE GROUPS, PREPARATION PROCESS THEREOF AND THEIR USE AS ANTIBACTERIALS, FUNGICIDES ANTIMICROBIALS

(30) Priorité: 22.12.2014 FR 1402990
(43) Date de publication de la demande: 01.11.2017
(73) Titulaire: CDP-Innovation, 69007 Lyon (FR); Centre National de la Recherche Scientifique (C.N.R.S.), 75016 Paris (FR); Fea, 06560 Opio (FR); Université Nice - Sophia Antipolis, 06103 Nice (FR)
(72) Inventeur: ADAO, Adrien, 06650 Opio (FR); DESMURS, Jean Roger, 06400 Cannes (FR); DUNACH CLINET, Isabel, 06270 Villeneuve Loubet (FR); MORIZUR, Vincent, 29870 Lannilis (FR); OLIVERO, Sandra, 06100 Nice (FR)
(74) Mandataire: Macquet, Christophe
(86) Numéro de dépôt international: PCT/FR2015/000239
(87) Numéro de publication internationale: WO 2016/102786

(56) Documents cités:
- EP-A1- 2 690 122
- EP-B1- 1 290 068
- WO-A2-2011/140416
- US-A1- 2011 195 341

## Description

La présente invention concerne de nouveaux polymères de formules I, II, III, IV, V, VI, VII, VIII, IX, X, XI, XII, XIII, XIV et XV contenant des fonctions sulfonates métalliques des éléments des colonnes (Ib), (IIb), (IIIa), (IIIb), (IVa), (IVb), (Va), (Vb), (VIb), (VIIb), (VIIIb), leurs procédés de préparation et leurs emplois comme antimicrobiens, antibactériens et antifongiques.

Il existe de nombreuses substances antibactériennes, antimicrobiennes, antifongiques présentées sous forme de solution ou de suspension. C'est le cas des ammoniums quaternaires utilisés dans de nombreux domaines tels que les produits d'entretien, les cosmétiques. Toutefois, ces composés présentent plusieurs inconvénients. L'antimicrobien dispersé dans le milieu se retrouve à la fin dans les biotopes et gêne le développement des bactéries ou champignons nécessaires aux biotransformations indispensables au monde vivant. Les antibactériens se retrouvent également dans les stations d'épuration où ils provoquent des dégradations de la flore bactérienne nécessaire au bon fonctionnement des stations d'épuration. De plus, les ammoniums quaternaires ne sont pas très efficaces en présence de composés organiques. Les ammoniums quaternaires sont désactivés par les savons et autres détergents anioniques, ainsi que les fibres de coton. De plus, ils ne doivent pas être utilisés dans une eau dure. On estime les niveaux efficaces à 200 ppm. Ils se dégradent à des températures supérieures à 100° C et perdent ainsi toute efficacité.

Un autre agent bactéricide est largement utilisé dans de nombreuses formulations comme par exemple les cosmétiques, des produits d'hygiène dentaire, des gels ; il s'agit du triclosan. Là encore des effets toxiques ont été observés par B. Gaume, N. Bourgougnon, S. Auzoux-Bordenave, B. Roig, B. Le Bot, G. Bedoux* (Comparative Biochemistry and Physiology, Part C, (2012), 156, 87-94).

Plus récemment, on a assisté au développement de nanoparticules, en particulier de nanoparticules d'argent, comme bactéricide. Pour pallier ces inconvénients, il a été développé récemment des bactéricides basés sur des sels nanoparticules d'argent. Ces antibactériens, qui sont entre autre utilisés en cosmétiques, présentent l'inconvénient de facilement passer les membranes et présentent un risque de toxicité pour les organismes vivants comme le mentionne les travaux de Soohee Kim, Doug-Young Ryu (J. Appl. Toxicol., (2013), 33, 78-89), de Fateme Mirzajani, Hossein Askari, Sara Hamzelou, Mohsen Farzaneh, Alireza Ghassempour (J. Appl. Toxicol., (2012), 32, 867-879) ou G. E. Batley*, J. K. Kirby, M. J. McLaughlin (Acc. Chem. Res., (2013), 46(3), 854-862). EP2690122 décrit des polymères de la famille des poly(aryl ether cétone)s contenant des fonctions sulfoniques ou sulfonates métalliques.

EP1290068 enseigne le traitement de PEEK avec d'acide chlorosulfonique et dimethylacetamide pour préparer des poly(aryl éther cétone)s contenant des fonctions sulfoniques.

Afin de répondre aux insuffisances des solutions existantes, la demanderesse a recherché d'autres solutions.

La présente invention concerne de nouveaux polymères de formules I, II, III, IV, V, VI, VII, VIII, IX, X, XI, XII, XIII, XIV et XV utilisés comme agent antibactériens, fongicides, antimicrobiens 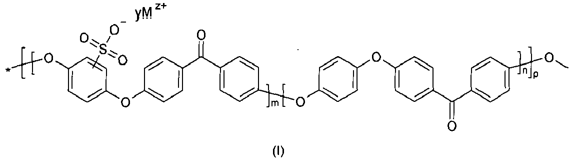 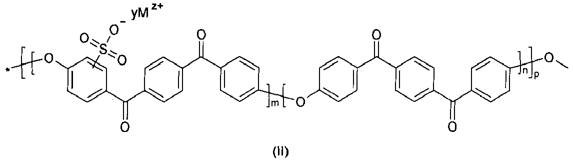 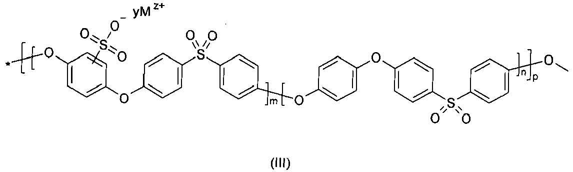 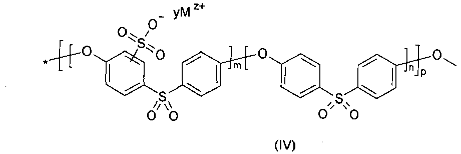 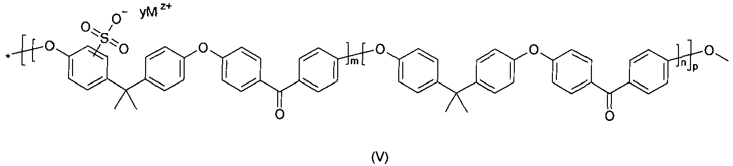 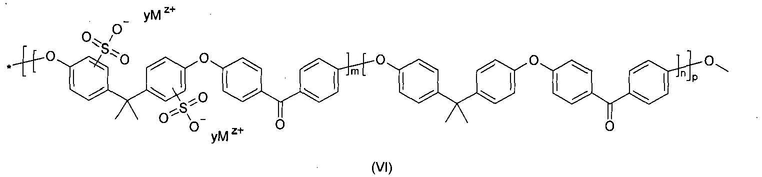 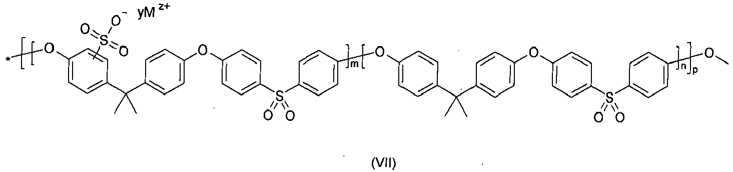 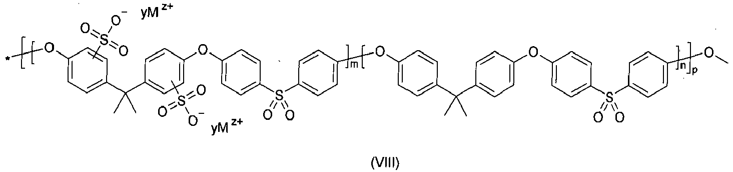 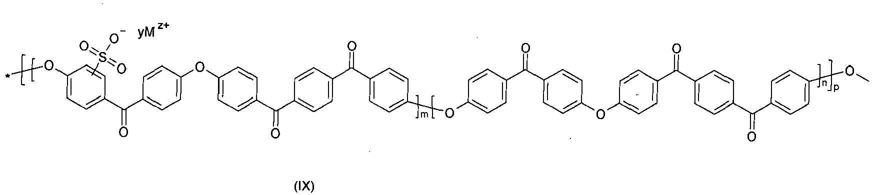 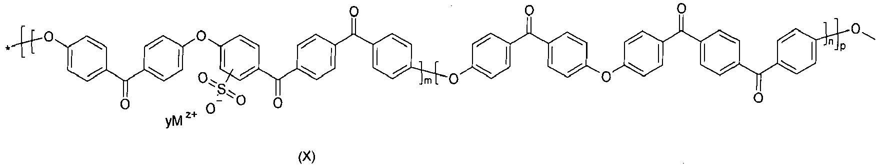 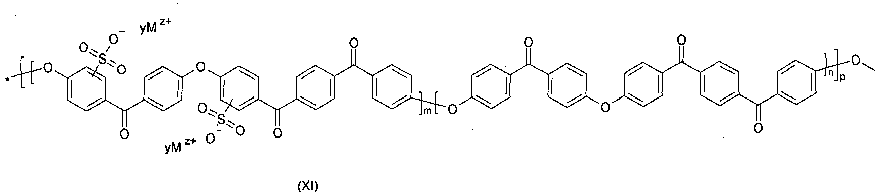 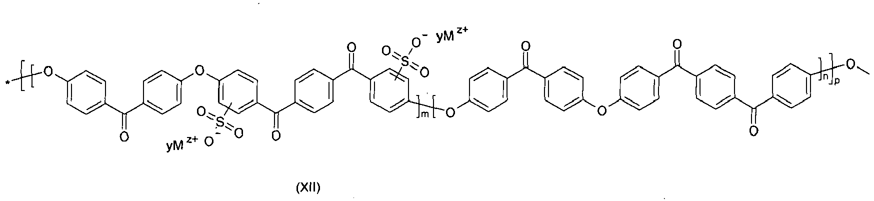 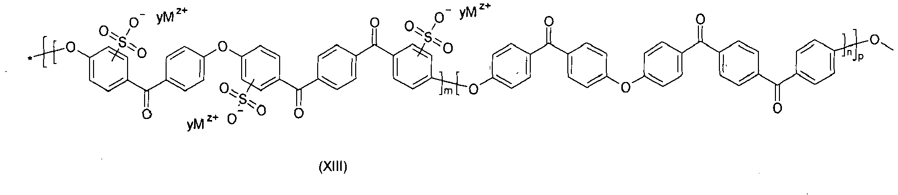 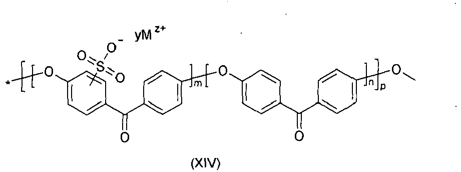 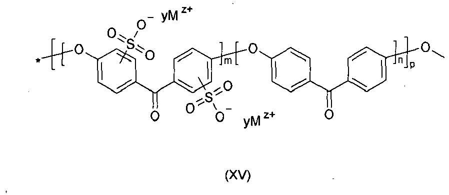 dans lesquelles :
- M^{z+} représente un cation correspondant à une forme oxydée des éléments des colonnes IB, IIB, IIIA, IIIB, IVA, IVB, VB, VIB, VIIB, VIIB,
- z représente un entier de 1 à 6 correspondant au degré d'oxydation du métal
- y représente un nombre entier ou fractionnaire égal à 1/z de manière à assurer la neutralité entre les charges négatives et positives
- m représente le pourcentage d'unités polymériques ayant un motif oxoaryle ou dioxoaryle possédant une fonction sulfonate. Ce pourcentage varie entre 0,1 et 100%, préférentiellement entre 1% et 100%.
- n représente le pourcentage d'unités polymériques ayant aucun motif oxoaryle ou dioxoaryle fonctionnalisé par un motif sulfonate. Ce pourcentage varie entre 0 et 99,9%, préférentiellement entre 0 et 99%.
- p représente le nombre d'unités polymériques du polymère ; p varie de 40 à 300, préférentiellement entre 60 et 200.

De manière préférentielle, M est choisi parmi les cations du titane, du zirconium, du vanadium, du chrome, du molybdène, du tungstène, du manganèse, du fer, du ruthénium, de l'osmium, du cobalt, du nickel, du palladium, du platine, du cuivre, de l'argent, de l'or, du zinc, de l'aluminium, du gallium, de l'indium, de l'étain, du bismuth.

De manière très préférentielle, M est choisi parmi les cations du titane, du cuivre, de l'argent, du zinc, de l'or.

Les polymères de formules I, II, III, IV, V, VI, VII, VIII, IX, X, XI, XII, XIII, XIV et XV peuvent être obtenus
1) en réalisant dans une première étape la chlorosulfonation d'un polymère de formules XVI, XVII, XVIII, XIX, XX, XXI, XXII, XXIII par un mélange d'acide chlorosulfonique, de chlorure de thionyle et d'un amide selon un mode opératoire optimisé. 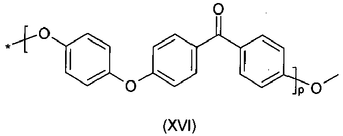 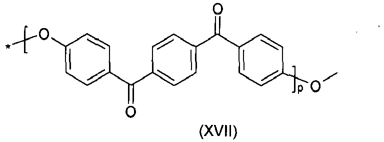 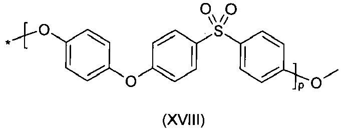 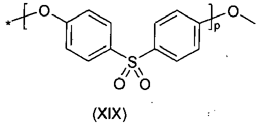 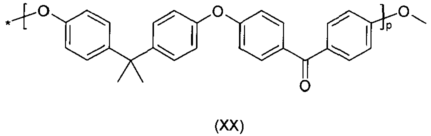 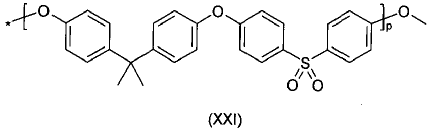 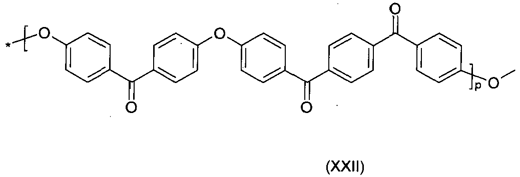 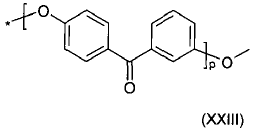 dans lesquelles
   p représente le nombre d'unités polymériques du polymère; p varie de 40 à 300, préférentiellement entre 60 et 200,
   pour obtenir les polymères de formules XXIV, XXV, XXVI, XVII, XXVIII, XIX, XXX, XXXI, XXXII, XXXIII, XXXIV, XXXV, XXXVI, XXXVII et XXXVIII 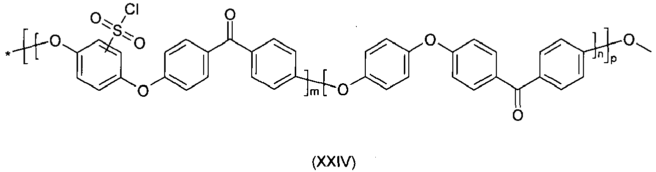 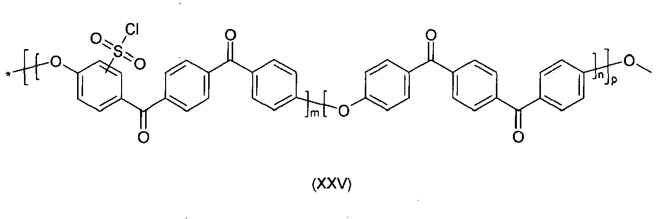 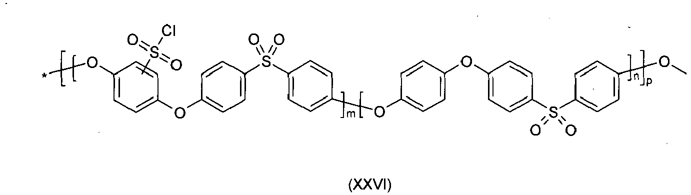 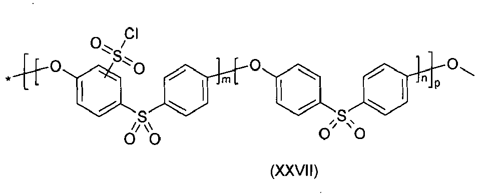 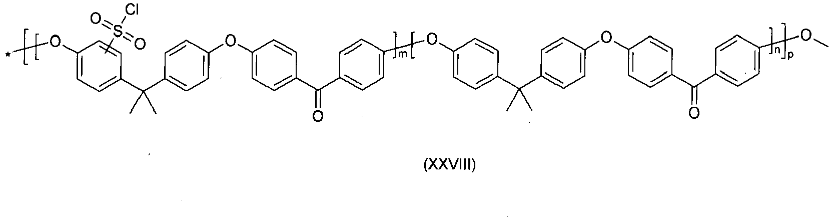 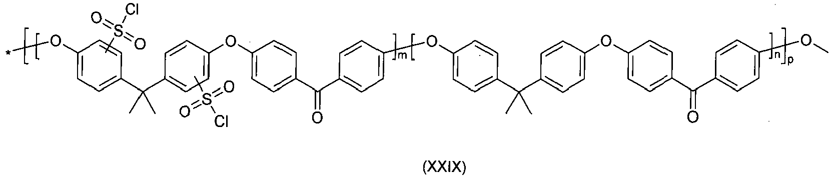 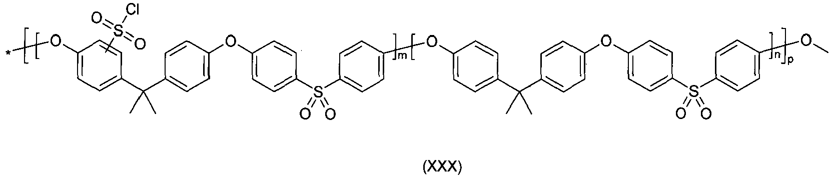 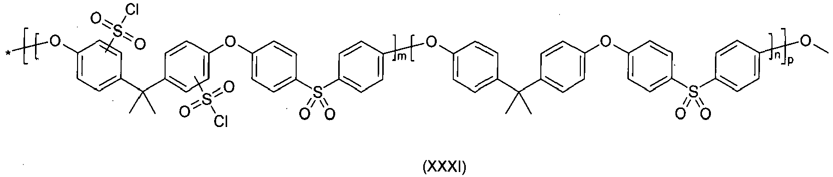 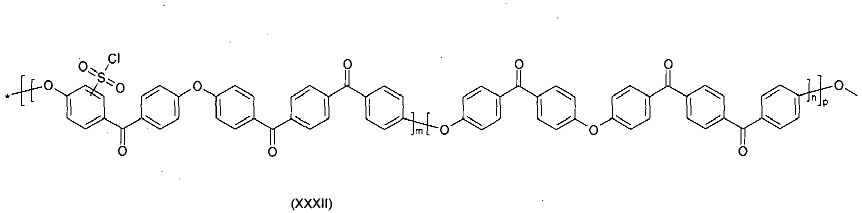 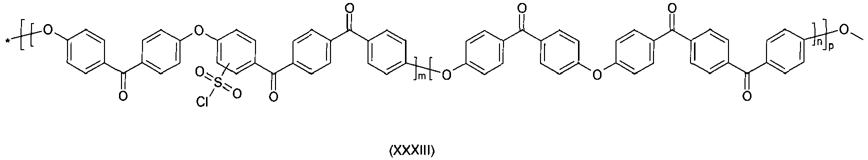 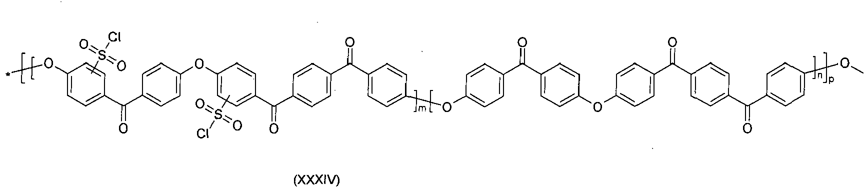 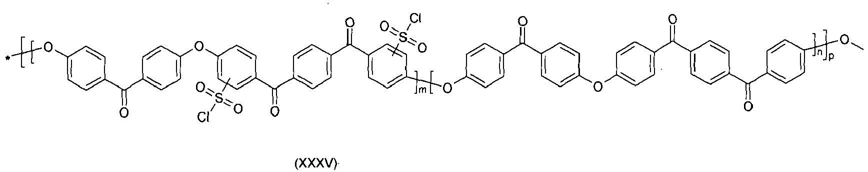 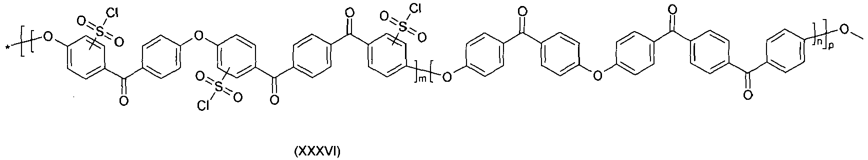 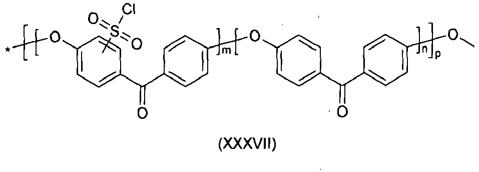 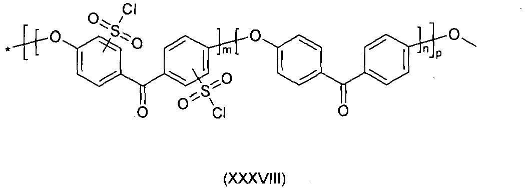 dans lesquelles :
   - m représente le pourcentage d'unités polymériques ayant un motif oxoaryle ou dioxoaryle fonctionnalisé par un groupement chlorure de sulfonyle. Ce pourcentage varie entre 0,1 et 100%, préférentiellement entre 1 et 100%.
   - n représente le pourcentage d'unités polymériques ayant un motif oxoaryle ou dioxoaryle non fonctionnalisé par un groupement chlorure de sulfonyle. Ce pourcentage varie entre 0 et 30%, préférentiellement entre 0 et 99%,
   - p représente le nombre d'unités polymériques du polymère; p varie de 40 à 300, préférentiellement entre 60 et 200.

Les polymères de départ XVI, XV, XVI, XVII, XVIII, XIX, XX, XXI, XXII, XXIII sont des produits commerciaux. Le polymère de formule XVI est connu commercialement sous le nom de poly(éther éther cétone) ou poly ether ether ketone ou PEEK, 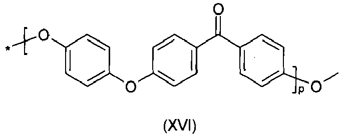 le polymère XVII est connu commercialement sous le nom de poly(éther cétone cétone) ou poly ether ketone ketone ou PEKK 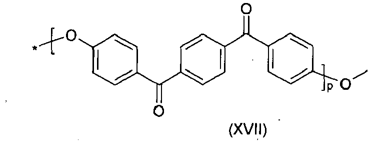 le polymère XVIII est connu commercialement sous le nom de poly(ether ether sulfone) ou PEES 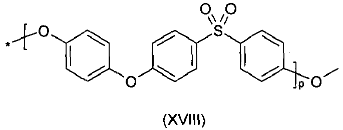 le polymère XIX est connu commercialement sous le nom de poly(ether sulfone) ou PES 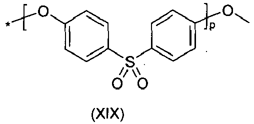 le polymère XX fait partie de la famille des poly(arène éther cétone) et est connu commercialement sous le nom de poly(bisphénol A PAEK) 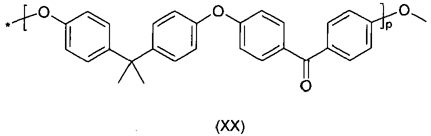 le polymère XXI fait partie de la famille des poly(arène éther sulfone) et est connu commercialement sous le nom de poly(bisphénol A PAES) 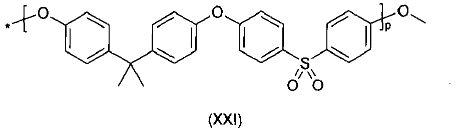 le polymère XXII est connu commercialement sous le nom de poly(éther cétone éther cétone cétone) ou poly(ether ketone ether ketone ketone) ou PEKEKK 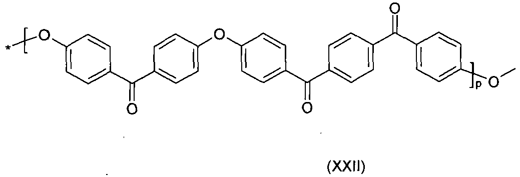 le polymère XXIII est connu commercialement sous le nom de poly(éther cétone) ou poly(ether ketone) ou PEK

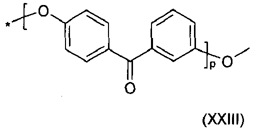

Cette liste de polymères n'est pas limitative puisqu'il existe un grand nombre d'autres polymères commercialement disponibles dans les familles des poly(aryl éther cétone), poly(aryl éther sulfone). Selon l'invention, les polymères préférés en raison de leur grande disponibilité sont le PEEK, le PEK, le PES, le PEKK et le PEKEKK.

La chlorosulfonation est réalisée à une température comprise entre 0° et 80° C en introduisant par rapport aux motifs oxoaryles ou dioxoaryles 0,1 à 10 équivalents d'acide chlorosulfonique, 0,1 à 30 équivalents de chlorure de thionyle, 0,1 à 10 équivalents d'un amide préférentiellement le diméthylformamide, avec ou sans solvant. Les solvants préférés selon l'invention sont le THF, le méthylTHF, le dichlorométhane, le dichloroéthane. De manière préférentille, la chlorosulfonation est réalisée à une température comprise entre 0 et 80 °C par un mélange de 0,1 à 5 équivalents d'acide chlorosulfonique, 0,1 à 15 équivalents de chlorure de thionyle en présence de 0,1 à 5 équivalents d'un amide par rapport aux motifs oxoaryles ou dioxoaryles à chlorosulfoner pour obtenir des taux de chlorosulfonation compris entre 0,1 et 50%.

La chlorosulfonation de certains polymères peut conduire à des mélanges de nombreux isomères. Cela est particulièrement vrai pour les polymères XXII ou PEKEKK. Les polymères chlorosulfonés XXXII, XXXIII, XXXIV, XXXV et XXXVI sont donnés à titre d'exemple. D'autres isomères peuvent être formés durant la chlorosulfonation.
2) dans une deuxième étape, on fait réagir sur les polymères de formules XXIV, XXV, XXVI, XVII, XXVIII, XIX, XXX, XXXI, XXXII, XXXIII, XXXIV, XXXV, XXXVI, XXXVII et XXXVIII de l'eau ou une solution aqueuse à une température comprise entre 60 et 140 °C, préférentiellement entre 80 et 120 °C pour obtenir les polymères de formules XIL, XL, XLI, XLII, XLIII, XLIV, XLV, XLVI, XLVII, XLVIII, XLIX, L, LI, LII, LIII, 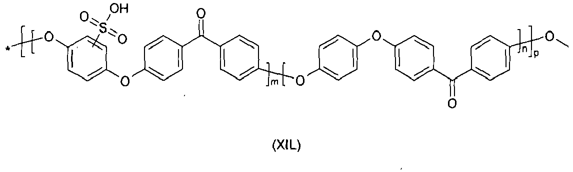 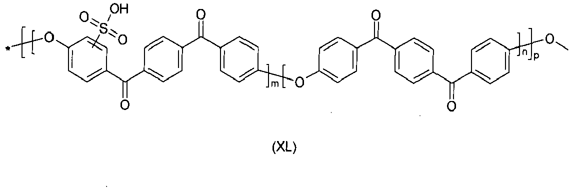 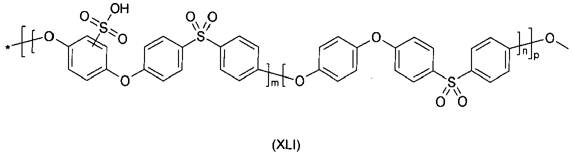 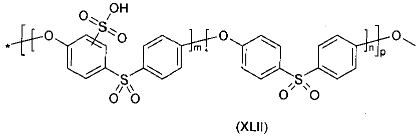 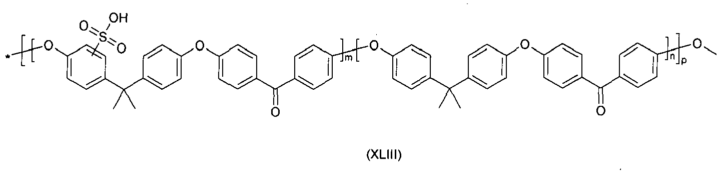 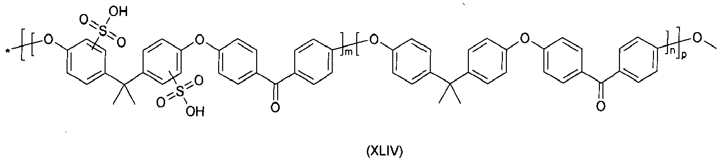 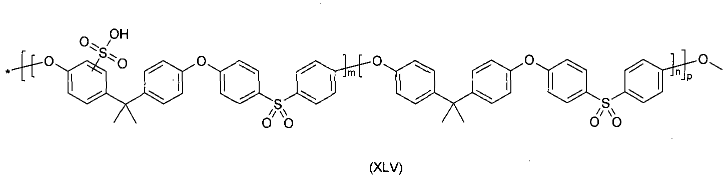 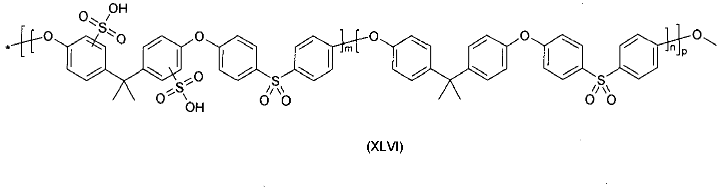 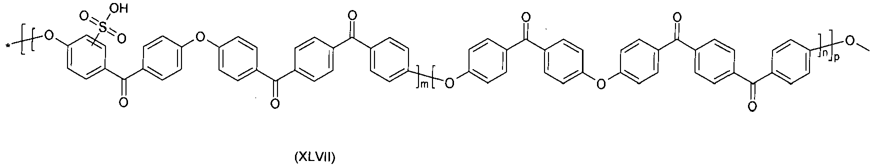 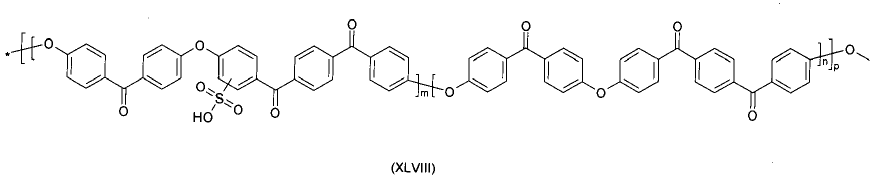 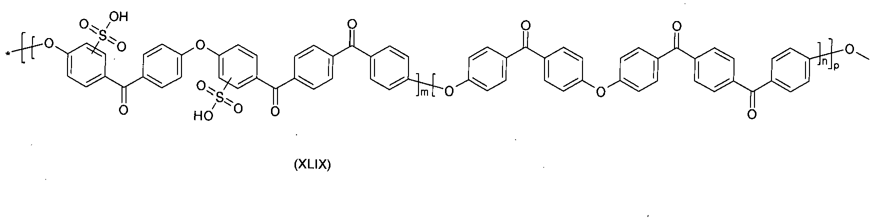 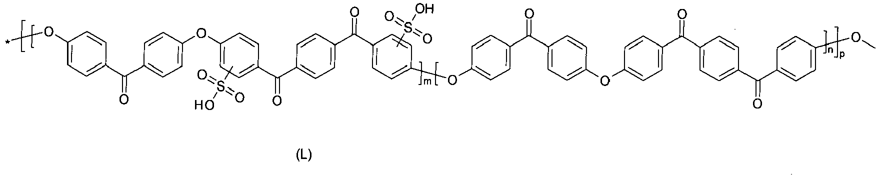 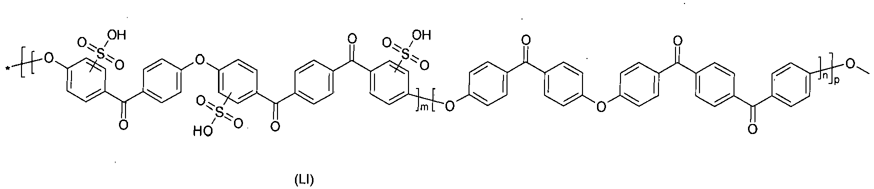 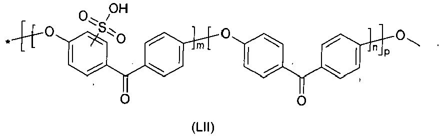 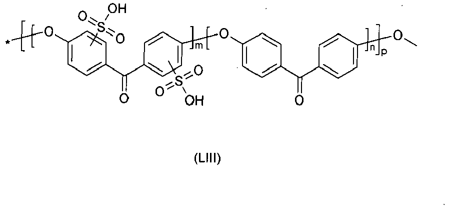 dans lesquelles :
   - m représente le pourcentage d'unités polymériques ayant un motif oxoaryle ou dioxoaryle fonctionnalisé possédant une fonction sulfonique. Ce pourcentage varie entre 0,1 et 100%, préférentiellement entre 1 et 100%
   - n représente le pourcentage d'unités polymériques ayant un motif oxoaryle ou dioxoaryle non fonctionnalisé par une fonction sulfonique. Ce pourcentage varie entre 0 et 99,9%, préférentiellement entre 0 et 99%
   - p représente le nombre d'unités polymériques du polymère; p varie de 40 à 300, préférentiellement entre 60 et 200.

Les solvants utilisés pour la réaction d'hydrolyse par l'eau avec les polymères chlorosulfonés de formules XXIV, XXV, XXVI, XVII, XXVIII, XIX, XXX, XXXI, XXXII, XXXIII, XXXIV, XXXV, XXXVI, XXXVII et XXXVIII sont des solvants aprotiques polaires. Les solvants préférés sont l'eau ou des mélanges d'eau avec le THF, le méthylTHF, le dioxane, le dichlorométhane, le *N,N*-diméthylformamide, la N-méthylpyrrolidone, le diméthylsulfoxyde, l'éthanol, l'isopropanol.

L'évaporation de l'eau et/ou des solvants permet d'obtenir les polymères XIL, XL, XLI, XLII, XLIII, XLIV, XLV, XLVI, XLVII, XLVIII, XLIX, L, LI, LII, LIII à l'état solide.
3) dans une troisième étape, lorsque l'on souhaite avoir un cation métallique, on fait réagir dans une première variante les polymères de formules XIL, XL, XLI, XLII, XLIII, XLIV, XLV, XLVI, XLVII, XLVIII, XLIX, L, LI, LII et LIII avec un sel métallique du métal M ou directement le métal M au degré d'oxydation 0 en présence d'ultrason.

Parmi les sels métalliques utilisables dans la présente invention, nous citerons les chlorures, les bromures, les carbonates, les alkylcarboxylates tels que les acétates, les propionates, les arylcarboxylates tels que les benzoates, les acétylacétates, les acétylacétonates, les alcoolates, les phénates.

De manière préférentielle, on préférera utiliser des sels d'acides faibles dont le pKa est supérieur à 0 comme les carbonates, les acétates, les propionates, les benzoates, les alcoolates, les acétylacétates, les acétylacétonates.

Dans une seconde variante, on fait réagir les polymères de formules XIL, XL, XLI, XLII, XLIII, XLIV, XLV, XLVI, XLVII, XLVIII, XLIX, L, LI, LII, LIII avec un métal au degré 0 en présence d'ultrason selon la technique décrite dans le brevet FR 2963003. Les métaux préférés pour cette seconde variante sont le bismuth, l'indium, le fer, le cuivre, le gadolinium, le samarium.

La réaction de formation des sels est réalisée de préférence dans un solvant où le polymère est soluble ou partiellement soluble. Les solvants préférentiels sont l'eau, le THF, le méthylTHF, le dioxane, le dichlorométhane, le *N,N*-diméthylformamide, la *N-*méthylpyrrolidone, le diméthylsulfoxyde, l'éthanol, l'isopropanol, le nitrométhane. De manière préférentielle, on utilise l'eau comme solvant.

L'évaporation de l'eau ou du solvant permet d'obtenir les polymère de formules I, II, III, IV, V, VI, VII, VIII, IX, X, XI, XII, XIII, XIV et XV à l'état solide.

Les polymères de formules I, II, III, IV, V, VI, VII, VIII, IX, X, XI, XII, XIII, XIV et XV dans lesquelles M est un métal, sont des composés bactéricides, fongicides et antimicrobiens.

Les polymères de formules I, II, III, IV, V, VI, VII, VIII, IX, X, XI, XII, XIII, XIV et XV dans lesquelles M^{z+} correspond aux cations argent et/ou cuivre présentent de très fortes activités antibactérienne, antifongiques et antimicrobiennes.

De manière surprenante, il a été observé que les polymères de formules XIL, XL, XLI, XLII, XLIII, XLIV, XLV, XLVI, XLVII, XLVIII, XLIX, L, LI, LII, LIII sont également fongicides, antimicrobiens et antibactériens. En particulier, une forte activité a été observée vis-à-vis de Staphilococcaus Aureus et Pseudomonas aeruginosa.

Les polymères de formules I, II, III, IV, V, VI, VII, VIII, IX, X, XI, XII, XIII, XIV et XV peuvent être facilement incorporés aux polymères non fonctionnalisés de formule XVI, XVII, XVIII, XIX, XX, XXI, XXII, XXIII pour obtenir des matériaux bactéricides ou fongicides. Cela est d'autant plus important que les polymères de formules XVI, XVII, XVIII, XIX, XX, XXI, XXII, XXIII sont souvent utilisés dans le domaine médical ou de l'analyse pour faire des implants, des tubulures.

Il est aussi possible de réaliser des polymères de formules I, II, III, IV, V, VI, VII, VIII, IX, X, XI, XII, XIII, XIV et XV dans lesquelles le taux de fonctionnalisation m est faible, de 0,1 à 5%, et d'obtenir des matériaux dont les propriétés physico-chimiques, rhéologiques sont très proches de celles des polymères de départ de formule XVI, XVII, XVIII, XIX, XX, XXI, XXII, XXIII. Cela est très intéressant pour la mise en oeuvre des polymères par les plasturgistes.

Les exemples figurant ci-après sont présentés à titre illustratif et non limitatif de l'objet de la présente invention.

### Exemple 1 - Chlorosulfonation du PEEK (XVI). Préparation du polymère XXIV

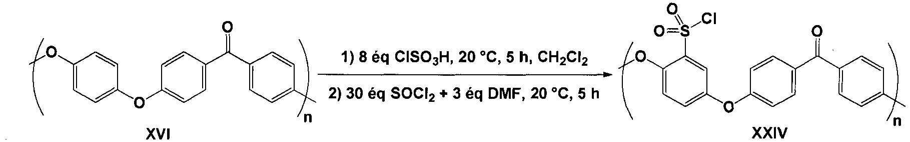

Sous atmosphère d'azote, dans un réacteur en verre cylindrique, on pèse directement 1,0 g de PEEK (XVI), puis on ajoute 160 mL de dichlorométhane distillé de manière à avoir une concentration molaire en PEEK (XVI) de 0,0217 M. Tous les solvants utilisés lors de ces synthèses ont été distillés, stockés et prélevés sous atmosphère d'azote.

L'acide chlorosulfonique (3,24 g, 8 équivalents par rapport au nombre d'unités polymériques du PEEK (XVI)) est introduit à l'aide d'une seringue (1,85 mL) en prenant soin de manipuler sous flux d'azote. Le mélange réactionnel est agité à 20 °C pendant 5 h. En fin de réaction, on observe la formation d'un composé visqueux orange. Le surnageant est éliminé en prenant soin de manipuler sous flux d'azote.

On introduit ensuite le chlorure de thionyle(12,29 g, 30 équivalents par rapport au nombre d'unités polymériques du PEEK (XVI)) à l'aide d'une seringue (7,50 mL) en prenant soin de manipuler sous flux d'azote. Puis on ajoute le *N,N*-diméthylformamide (0,76 g, 3 équivalents par rapport au nombre d'unités polymériques du PEEK (XVI)) à l'aide d'une seringue (0,81 mL) en prenant soin de manipuler sous flux d'azote. Le mélange réactionnel est de nouveau agité à 20 °C pendant 5 h, puis on ajoute 40 mL de THF distillé. En fin de réaction, on observe une solution orange.

La solution orange est précipitée dans du propan-2-ol (250 mL), un précipité blanc se forme. Le solide est filtré, puis lavé avec 2 fois 50 mL de propan-2-ol et 2 fois 50 mL d'acétonitrile, puis séché pendant une nuit sous vide (1.10⁻² mbar).

Le spectre RMN ¹H réalisé dans le DMSO-D6 (¹H NMR (200 MHz, DMSO) δ 7,92 - 7,69 (m, 4H), 7,50 (d, *J* = 2,7 Hz, 1H), 7,36 - 6,83 (m, 6H)) confirme la structure attendue. L'intégration du pic à 7,50 ppm en RMN ¹H nous permet de connaître le taux de chlorosulfonation du PEEK (XVI). Le taux de chlorosulfonation des motifs dioxoaryle est de 100%.

Le rendement pondéral en polymère XXIV est de 98% par rapport au PEEK (XVI) engagé.

### Exemple 2-6 Préparation du polymère XXIV avec différents taux de fonctionnalisation

Selon le protocole décrit dans l'exemple 1 les polymères suivants ont été préparés :

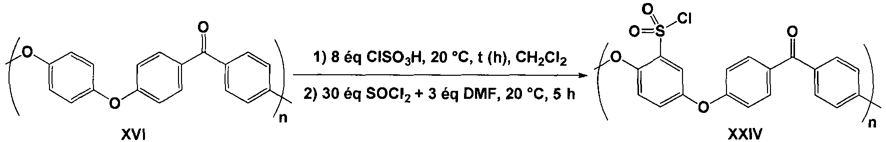

Les différences avec le protocole décrit dans l'exemple 1 sont :
- la masse de PEEK (XIV) de départ
- le temps de réaction de la première étape

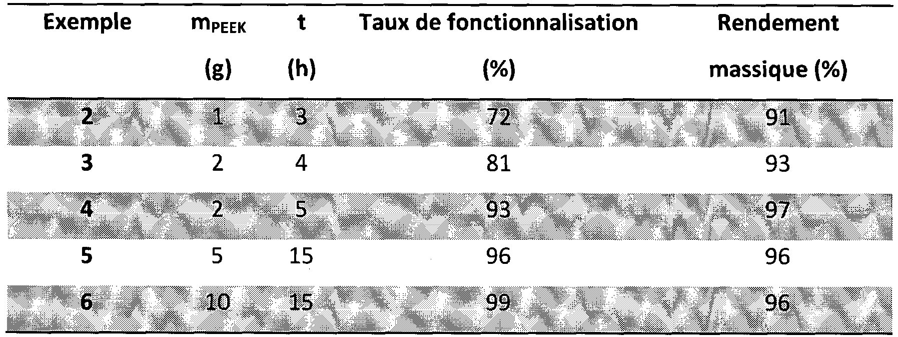

### Exemple 7 - Chlorosulfonation du PEES (XVIII). Préparation du polymère XXVI

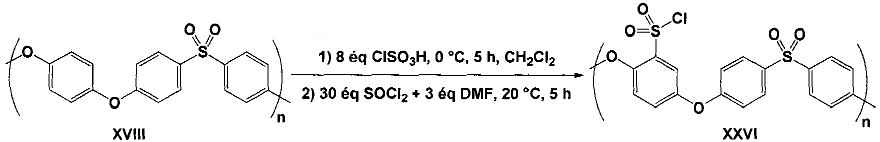

Sous atmosphère d'azote, dans un réacteur en verre cylindrique, on pèse directement 1,0 g de PEES (XVIII), puis on ajoute 160 mL de dichlorométhane distillé de manière à avoir une concentration molaire en PEES (XVIII) de 0,019 M. Tous les solvants utilisés lors de ces synthèses ont été distillés, stockés et prélevés sous atmosphère d'azote.

L'acide chlorosulfonique (2,88 g, 8 équivalents par rapport au nombre d'unités polymériques du PEES (XVIII)) est introduit à l'aide d'une seringue (1,64 mL) en prenant soin de manipuler sous flux d'azote. Le mélange réactionnel est agité à 0 °C pendant 5 h. En fin de réaction, on observe la formation d'un composé visqueux brun. Le surnageant est éliminé en prenant soin de manipuler sous flux d'azote.

On introduit ensuite le chlorure de thionyle(10, 93 g, 30 équivalents par rapport au nombre d'unités polymériques du PEES (XVIII)) à l'aide d'une seringue (6,6 mL) en prenant soin de manipuler sous flux d'azote. Puis on ajoute le *N,N*-diméthylformamide (0,76 g, 3 équivalents par rapport au nombre d'unités polymériques du PEES (XVIII)) à l'aide d'une seringue (0,68 mL) en prenant soin de manipuler sous flux d'azote. Le mélange réactionnel est de nouveau agité à 20 °C pendant 5 h, puis on ajoute 15 mL de CH₂Cl₂ distillé. En fin de réaction, on observe une solution brune.

La solution brune est précipitée dans du propan-2-ol (250 mL), un précipité blanc se forme. Le solide est filtré, puis lavé avec 2 fois 50 mL de propan-2-ol et 2 fois 50 mL d'acétonitrile, puis séché pendant une nuit sous vide (1.10⁻² mbar) .

Le spectre RMN ¹H réalisé dans le DMSO-D6 (¹H NMR (200 MHz) δ 7,91 (ddd, J = 18,6, 8,8, 2,9 Hz, 4H), 7,44 (s, 1H), 7,18 (d, J = 7,2 Hz, 4H), 6,99 (d, J = 7,3 Hz, 2H) confirme la structure attendue.

L'intégration du pic à 7,44 ppm en RMN ¹H nous permet de connaître le taux de chlorosulfonation du PEES (XVIII). Le taux de chlorosulfonation des motifs dioxoaryle est de 100%.

Le rendement pondéral en polymère XXVI est de 93 % par rapport au polymère XVIII engagé.

### Exemple 8 - Chlorosulfonation du PES (XIX). Préparation du polymère XXVII

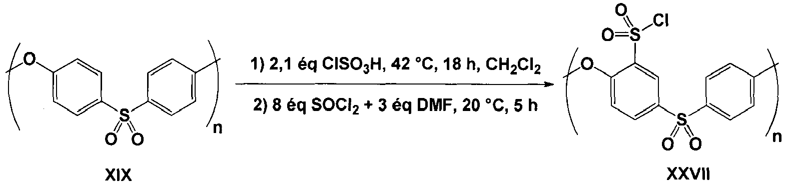

Sous atmosphère d'azote, dans un réacteur en verre cylindrique, on pèse directement 0,5 g de PES (XIX), puis on ajoute 80 mL de dichlorométhane distillé de manière à avoir une concentration molaire en PES (XIX) de 0,027 M, après 1 h sous agitation à 20 °C, le PES (XIX) est solubilisé. Tous les solvants utilisés lors de ces synthèses ont été distillés, stockés et prélevés sous atmosphère d'azote.

L'acide chlorosulfonique (0,527 g, 2,1 équivalents par rapport au nombre d'unités polymériques du PES (XIX)) est introduit à l'aide d'une seringue (0,30 mL) en prenant soin de manipuler sous flux d'azote. Le mélange réactionnel est agité à 42 °C pendant 18 h. En fin de réaction, on observe la formation d'un composé visqueux jaune. Le surnageant est éliminé en prenant soin de manipuler sous flux d'azote.

On introduit ensuite le chlorure de thionyle(2,03 g, 8 équivalents par rapport au nombre d'unités polymériques du PES (XIX)) à l'aide d'une seringue (1,24 mL) en prenant soin de manipuler sous flux d'azote. Puis on ajoute le *N,N*-diméthylformamide (0,47 g, 3 équivalents par rapport au nombre d'unités polymériques du PES (XIX)) à l'aide d'une seringue (0,50 mL) en prenant soin de manipuler sous flux d'azote. Le mélange réactionnel est de nouveau agité à 20 °C pendant 5 h, puis on ajoute 15 mL de CH₂Cl₂ distillé. En fin de réaction, on observe une solution jaune.

La solution jaune est précipitée dans du propan-2-ol (80 mL), un précipité blanc se forme. Le solide est filtré, puis lavé avec 2 fois 20 mL de propan-2-ol et 3 fois 20 mL d'acétonitrile, puis séché pendant une nuit sous vide (1.10⁻² mbar) .

Le spectre RMN 1H réalisé dans le DMSO-D6 (¹H NMR (200 MHz) δ 8,29 (s, 1H), 7,92 (s, 3H), 7,19 (s, 3H)) confirme la structure attendue. L'intégration du pic à 8,29 ppm en RMN ¹H nous permet de connaître le taux de chlorosulfonation du PES (XIX). Le taux de chlorosulfonation des motifs oxoaryle est de 100%.
Le rendement pondéral en polymère XXVII est de 98% par rapport au polymère XIX engagé.

### Exemple 9- Hydrolyse du polymère XXIV : Préparation du polymère XIL

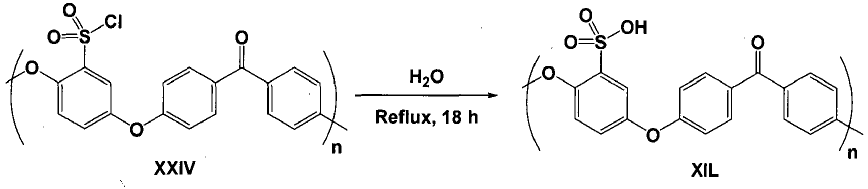

Dans un réacteur en verre cylindrique, on pèse directement 10,0 g de polymère XXIV, puis on ajoute 250 mL d'eau de manière à avoir une concentration molaire en PEEK de 0,104 M.

Le mélange réactionnel est agité à 100 °C pendant 18 h. En fin de réaction, on observe la solubilisation du polymère. L'eau est évaporée à l'aide d'un évaporateur rotatif. Le solide obtenu est séché pendant une nuit sous vide (1.10⁻² mbar). Après l'évaporation du solvant on obtient un solide doré.

Le spectre RMN ¹H est réalisé dans le DMSO-D6 (RMN ¹H (200 MHz, DMSO) δ 8,07 - 7,60 (m, 4H), 7,51 (d, J = 2,7 Hz, 1H), 7,35 - 6,82 (m, 6H)) confirme la structure attendue.

L'intégration du pic à 7,51 ppm en RMN ¹H nous permet de connaître le taux de sulfonation du PEEK (XVI).

Le rendement pondéral en polymère XIL est de 99% par rapport au polymère XXIV engagé.

### Exemple 10 - Hydrolyse du polymère XXVI : Préparation du polymère XLI

Dans un réacteur en verre cylindrique, on pèse directement 1,0 g de XXVI, puis on ajoute 50 mL d'eau de manière à avoir une concentration molaire en PEES de 0,047 M.

Le mélange réactionnel est agité à 100 °C pendant 5 h. En fin de réaction, on observe la solubilisation du polymère. L'eau est évaporée à l'aide d'un évaporateur rotatif. Le solide obtenu est séché pendant une nuit sous vide (1.10⁻² mbar). Après l'évaporation du solvant on obtient un solide doré.

Le spectre RMN ¹H réalisé dans le DMSO-D6 (RMN ¹H (200 MHz, DMSO) δ 7,91 (ddd, *J* = 18,7, 8,7, 2,9 Hz, 4H), 7,44 (s, 1H), 7,18 (d, *J* = 6,9 Hz, 4H), 7,00 (d, *J* = 7,6 Hz, 2H)) confirme la structure attendue.

L'intégration du pic à 7,44 ppm en RMN ¹H nous permet de connaître le taux de sulfonation du PEES (XVIII).

Le rendement pondéral en polymère XLI est de 99% par rapport au polymère XXVI engagé.

### Exemple 11 - Hydrolyse du polymère XXVII : Préparation du polymère XLII

Dans un réacteur en verre cylindrique, on pèse directement 1,0 g de polymère XXVII, puis on ajoute 50 mL d'eau de manière à avoir une concentration molaire en PEES (XVIII) de 0,060 M.

Le mélange réactionnel est agité à 100 °C pendant 5 h. En fin de réaction, on observe la solubilisation du polymère. L'eau est évaporée à l'aide d'un évaporateur rotatif. Le solide obtenu est séché pendant une nuit sous vide (1.10⁻² mbar). Après l'évaporation du solvant on obtient un solide doré.

Le spectre RMN ¹H réalisé dans le DMSO-D6 (RMN ¹H (200 MHz, DMSO) δ 8,32 (s, 1H), 7,91 (s, 3H), 7,18 (s, 3H)) confirme la structure attendue. L'intégration du pic à 8,32 ppm en RMN ¹H nous permet de connaître le taux de sulfonation du PES (XIX).

Le rendement pondéral en polymère XLII est de 99% par rapport au polymère XXVII engagé.

### Exemple 12 - Métallation du polymère XIL avec de l'indium au degré 0: Préparation de I (avec M^{z+} = In³⁺)

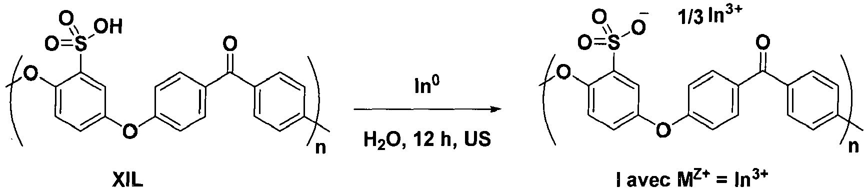

Dans un réacteur en verre cylindrique, on pèse directement 1 g de polymère XIL, puis on ajoute 30 mL d'eau de manière à avoir une concentration molaire en polymère de de 0,091 M.

L'indium (0,100 g) est introduit afin d'avoir des quantités stoechiométriques polymère-métal, c'est-à-dire dans ce cas 3 équivalents de polymère par atome d'indium. Le mélange réactionnel est mis dans une cuve à ultrasons à une fréquence de 35 KHz et à une puissance de 70 W à température ambiante pendant 12 h. En fin de réaction on observe un précipité gris-métal et tout l'indium est consommé. La solution est filtrée et le solide obtenu est séché pendant une nuit sous vide (1.10⁻² mbar).

Le spectre RMN ¹H dans le DMSO-D6 (RMN ¹H (200 MHz, DMSO) δ 7,90 - 7,68 (m, 4H), 7,50 (s, 1H), 7,32 - 6,95 (m, 6H)) et le spectre infrarouge sont réalisés. On observe sur le spectre RMN ¹H ainsi que sur le spectre infrarouge la disparition de la fonction OH du polymère ce qui est en accord avec la structure attendue.

Le rendement pondéral en I (avec M^{Z+} = In³⁺) est de 92% par rapport au polymère XIL engagé.

### Exemples 13 à 16 - Métallation du polymère XIL avec des métaux au degré 0. Préparation de I (avec M^{z+} = cation métallique)

Selon le protocole décrit dans l'exemple 12 les polymères suivants ont été préparés :

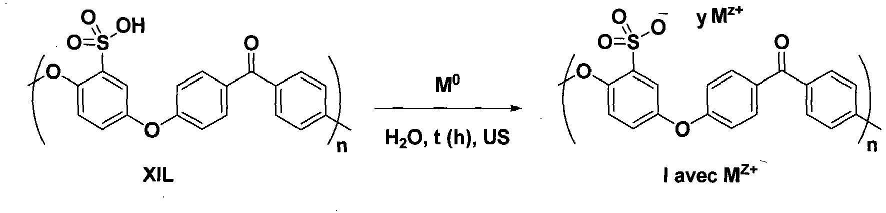

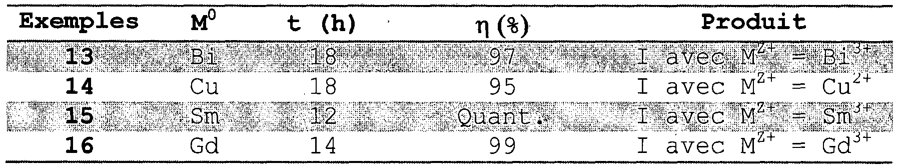

### Exemple 17 - Métallation du polymère XLII avec du cuivre au degré 0. Préparation de III (avec M^{Z+} = Cu²⁺)

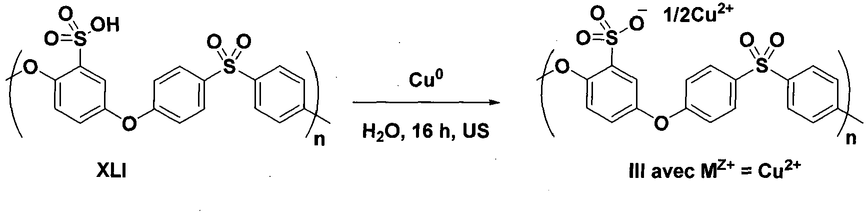

Dans un réacteur en verre cylindrique, on pèse directement 1 g du polymère XLI, puis on ajoute 30 mL d'eau de manière à avoir une concentration molaire en polymère de 0,083 M.

Le cuivre (0,075 g) est introduit afin d'avoir des quantités stoechiométriques polymère-métal, c'est-à-dire dans ce cas 2 équivalents de polymère par atome de cuivre. Le mélange réactionnel est mis dans une cuve à ultrasons à une fréquence de 35 KHz et à une puissance de 70 W à température ambiante pendant 16 h. En fin de réaction on observe un précipité vert et tout le cuivre est consommé. La solution est filtrée et le solide obtenu est séché pendant une nuit sous vide (1.10⁻² mbar).

Le spectre RMN ¹H dans le DMSO-D6 (RMN ¹H (200 MHz, DMSO) δ 7,90 (ddd, J = 18,0, 8,6, 2,8 Hz, 4H), 7,44 (s, 1H), 7,19 (s, 4H), 6,90 (s, 2H)) et le spectre infrarouge sont réalisés. On observe sur le spectre RMN ¹H ainsi que sur le spectre infrarouge la disparition de la fonction OH du polymère ce qui est en accord avec la structure attendue. Le rendement pondéral en III (avec M^{Z+} = Cu²⁺) est de 94% par rapport au XLI engagé.

### Exemples 18 à 19 - Métallation du polymère XLI avec des métaux au degré 0. Préparation de III avec différents M^{Z+}

Selon le protocole décrit dans l'exemple 17, les polymères suivants ont été préparés :

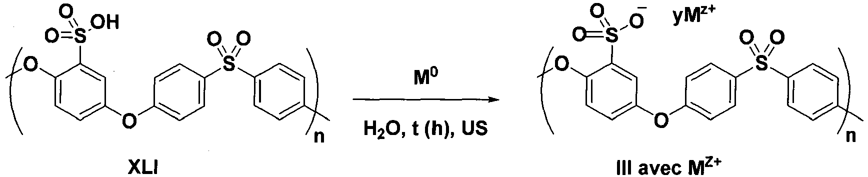

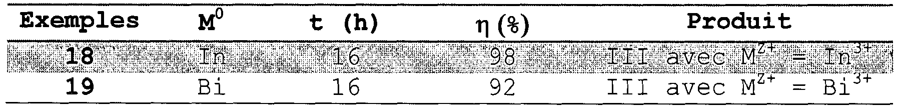

### Exemple 20 - Métallation du polymère XIL avec un sel de cuivre: Préparation de I (avec M^{Z+}=Cu²⁺)

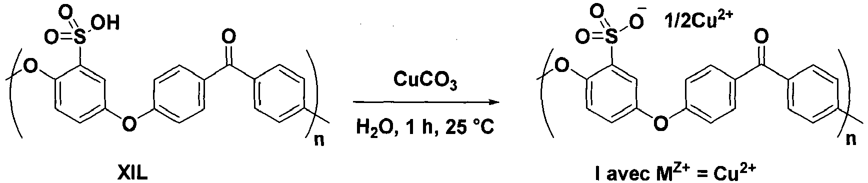

Dans un réacteur en verre cylindrique, on pèse directement 0,541 g de XIL, puis on ajoute 10 mL d'eau de manière à avoir une concentration molaire en polymère de de 0,147 M.

Le carbonate de cuivre (0,91 g) est introduit afin d'avoir des quantités stoechiométriques polymère-métal, c'est-à-dire dans ce cas 2 équivalents de polymère par atome de cuivre. Le mélange réactionnel est agité pendant 1 h à température ambiante. En fin de réaction on observe un précipité vert clair. La solution est évaporée et le solide obtenu est séché pendant une nuit sous vide (1.10⁻² mbar).

Le spectre RMN ¹H dans le DMSO-D6 (RMN ¹H (200 MHz, DMSO) δ 7,90 - 7,68 (m, 4H), 7,50 (s, 1H), 7,32 - 6,95 (m, 6H)) et le spectre infrarouge sont réalisés. On observe sur le spectre RMN ¹H ainsi que sur le spectre infrarouge la disparition de la fonction OH du polymère ce qui est en accord avec la structure attendue.

Le rendement pondéral en I (avec M^{z+} = Cu²⁺) est de 98% par rapport au polymère XIL engagé.

### Exemples 21 à 24 - Métallation du polymère XIL: Préparation de I (avec M^{Z+}).

Selon le protocole décrit dans l'exemple 20, les polymères suivants ont été préparés :

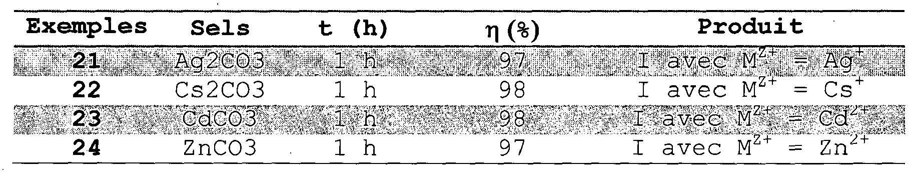

### Exemple 25 - Métallation du polymère XIL: Préparation du I (M^{Z+} avec In³⁺)

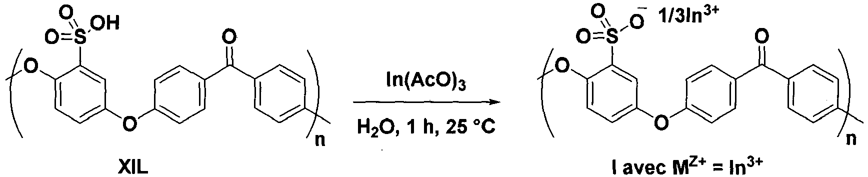

Dans un réacteur en verre cylindrique, on pèse directement 0,447 g du XIL, puis on ajoute 10 mL d'eau de manière à avoir une concentration molaire en polymère de de 0,121 M.

L'acétate d'indium (0,118 g) est introduit afin d'avoir des quantités stoechiométriques polymère-métal, c'est-à-dire dans ce cas 3 équivalents de polymère par atome d'indium. Le mélange réactionnel est agité pendant 1 h à température ambiante. En fin de réaction on observe un précipité gris clair. La solution est évaporée et le solide obtenu est séché pendant une nuit sous vide (1.10⁻² mbar).

Le spectre RMN ¹H dans le DMSO-D6 (RMN ¹H (200 MHz, DMSO) δ 7,90 - 7,68 (m, 4H), 7,50 (s, 1H), 7,32 - 6,95 (m, 6H)) et le spectre infrarouge sont réalisés. On observe sur le spectre RMN ¹H ainsi que sur le spectre infrarouge la disparition de la fonction OH du polymère ce qui est en accord avec la structure attendue.

Le rendement pondéral en I (M^{Z+} avec In³⁺) est de 97% par rapport au XIL engagé.

### Exemples 26 à 27 - Métallation du XIL: Préparation de I (avec différents M^{Z+}).

Selon le protocole décrit dans l'exemple 25, les polymères suivants ont été préparés :

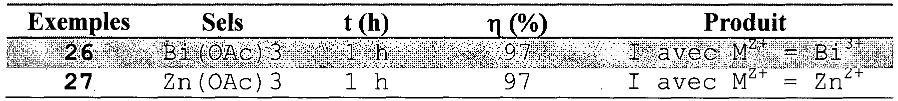

### Exemples 28 à 29 - Activité bactéricide des polymères I (avec M^{z+} = Ag⁺ et M^{z+} = Cu²⁺) sur la bactérie Pseudomonas aeruginosa.

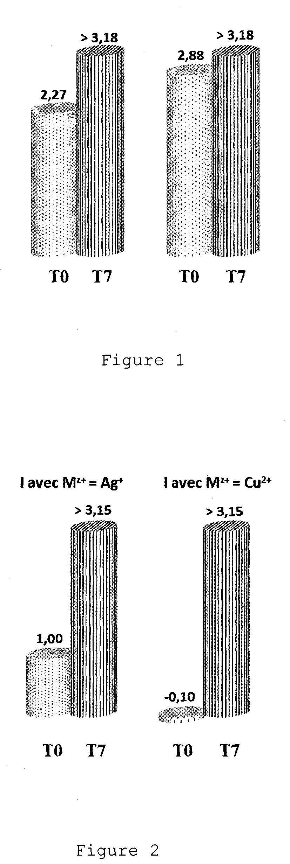

Le taux de réduction du nombre de *Pseudomonas aeruginosa* est représenté dans la figure 1 de la page 1/2.

Les valeurs indiquées sur cette figure sont exprimées en log de réduction des bactéries. Des valeurs ont été obtenues pour chaque sel à deux temps. T0 représente un temps de contact du polymère (I avec M^{z+} = Ag⁺ et M^{z+} = Cu²⁺) et de la bactérie inférieur à 1 min. T7 représente un temps de contact de 7 jours entre le polymère I (avec M^{z+} = Ag⁺ et M^{z+} = Cu²⁺) et la bactérie.

La valeur maximale mesurable avec cette méthode d'analyse est de log(réduction de bactéries)> 3,18.

### Exemples 30 à 31 - Activité bactéricide des polymères I (avec M^{z+} = Ag⁺ et M^{z+} = Cu²⁺) sur la bactérie Staphylococcus aureus.

Le taux de réduction du nombre de *Staphylococcus aureus* est représenté dans la figure 2 de la page 2/2.

Les valeurs indiquées sur cette figure sont exprimées en log de réduction des bactéries. Des valeurs ont été obtenues pour chaque sel à deux temps. T0 représente un temps de contact du polymère I (avec M^{z+} = Ag⁺ et M^{z+} = Cu²⁺) et de la bactérie inférieur à 1 min. T7 représente un temps de contact de 7 jours entre le polymère I (avec M^{z+} = Ag⁺ et M^{z+} = Cu²⁺) et la bactérie.

La valeur maximale mesurable avec cette méthode d'analyse est de log(réduction de bactéries)> 3,15.

### Exemples 32-1 à 32-49 - Activité anti-microbienne

Les Tableaux 2 et 3 ci-dessous présentent des résultats d'activité d'un polymère de formule générale (I) ou d'un mélange de polymère de formule générale (I) pour lesquels la valeur de p est comprise entre 40 et 60 sur différentes souches bactériennes ou différents microorganismes fongiques, à des temps t=0 et t=7 jours.

Ces résultats d'activité contenus dans ces Tableaux 2 et 3 sont exprimés en réduction logarithmique du nombre de micro-organismes aux temps précités. Ils sont à comparer avec les résultats obtenus pour le phénoxyéthanol, qui sont présentés dans le Tableau 1 ci-dessous :

**Tableau 1 :**

| Composé | Microorganismes | Réduction logarithmique à t=0 | Réduction logarithmique à t=7 jours |
|---|---|---|---|
| Phénoxyethanol | *Pseudomonas aeruginosas* | 1,9 | > 3,18 |
| | *Staphylococcus aureus* | -0,18 | > 3,15 |

Le mode opératoire est le suivant. On charge une quantité connue de polymère à analyser comprise entre 10 et 25 mg dans une boîte de culture. La boîte de culture est ensuite contaminée par un dépôt d'une suspension de microorganismes en ciblant une contamination finale de l'ordre de 10⁶ UFC/mL. Un échantillon est ensuite prélevé. Il est soit analysé immédiatement (t=0) ou conservé pendant 7 jours à 24°C +/-1°C (t=7). A chaque point de contrôle, le dénombrement est réalisé selon la procédure suivante. On ajoute 1 mL de bouillon TSB capitol IV dans un tube dans lequel on a placé l'échantillon prélevé. On ajoute 10 µL en gélose Trypticase soja pour Pseudomonas aeruginosas, Staphylococcus aureus, Escherichia coli et 100 µL de gélose Sabouraud pour les microorganismes fongiques : Candida albicans et Asperigillus brasiliensis. On incube le volume restant 48 h à 37 °C en vue d'un enrichissement du bouillon. 100 µL du bouillon enrichi sont ensuite isolés et incubés pendant 48 h à 37 °C. Les microorganismes sont ensuite comptés.

**Tableau 2**

| POLYMERE | | | | |
|---|---|---|---|---|
| 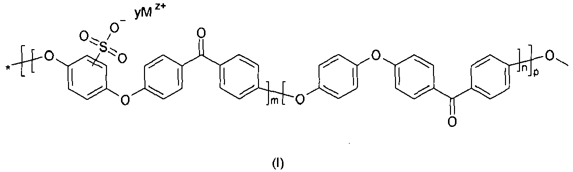 | | | | |

| Exemples | Sel métallique M^{y+} | Microorganismes | Réduction logarithmique à t=0 | Réduction logarithmique à t=7 jours |
|---|---|---|---|---|
| 32-1 | H⁺ m= 100% | *Pseudomonas aeruginosas* | > 3,18 | > 3,18 |
| 32-2 | | *Staphylococcus aureus* | 0,11 | > 3,15 |
| 32-3 | Cl m= 100% | *Pseudomonas aeruginosas* | -0,03 | > 3,18 |
| 32-4 | | *Staphylococcus aureus* | -0,14 | > 3,15 |
| 32-5 | Ag⁺ m= 100% | *Pseudomonas aeruginosas* | 2,27 | > 3,18 |
| 32-6 | | *Staphylococcus aureus* | 1,00 | > 3,15 |
| 32-7 | Cu²⁺ m= 100% | *Pseudomonas aeruginosas* | 2,88 | > 3,18 |
| 32-8 | | *Staphylococcus aureus* | -0,10 | > 3,15 |
| 32-9 | Fe²⁺ m= 100% | *Pseudomonas aeruginosas* | 2,96 | > 3,44 |
| 32-10 | | *Staphylococcus aureus* | 1,10 | > 3,18 |
| 32-11 | | *Escherichia coli* | 0,56 | >3,36 |
| 32-12 | | *Candida albicans* | -0,25 | >3,23 |
| 32-13 | | *Asperigillus brasiliensis* | -0,23 | 3,25 |
| 32-14 | Na⁺ m= 100% | *Pseudomonas aeruginosas* | 1,11 | > 3,44 |
| 32-15 | | *Staphylococcus aureus* | 0,43 | 2,88 |
| 32-16 | In³⁺ m= 100% | *Pseudomonas aeruginosas* | 0,46 | > 3,44 |
| 32-17 | | *Staphylococcus aureus* | 0,58 | > 3,18 |
| 32-18 | Al³⁺ m= 100% | *Pseudomonas aeruginosas* | -0,19 | > 3,44 |
| 32-19 | | *Staphylococcus aureus* | 0,11 | > 3,18 |
| 32-20 | Fe³⁺ m= 100% | *Pseudomonas aeruginosas* | 0,62 | > 3,52 |
| 32-21 | | *Staphylococcus aureus* | < 0,07 | > 3,54 |
| 32-22 | Zn²⁺ m= 100% | *Pseudomonas aeruginosas* | 1,62 | > 3,52 |
| 32-23 | | *Staphylococcus aureus* | 0,25 | > 3,54 |
| 32-24 | Fonctionnalisé m=33 % H⁺ | *Pseudomonas aeruginosas* | 0,69 | > 3,52 |
| 32-25 | | *Staphylococcus aureus* | 0,71 | > 3,54 |
| 32-26 | Ag+ m= 33 % | *Pseudomonas aeruginosas* | 2,52 | > 3,52 |
| 32-27 | | *Staphylococcus aureus* | 0,57 | > 3,54 |
| 32-28 | Na⁺/Ag⁺ (9/1) | *Pseudomonas aeruginosas* | 0,13 | > 3,44 |
| 32-29 | | *Staphylococcus aureus* | -0,09 | > 3,18 |
| 32-30 | Na⁺/Ag⁺ (1/1) | *Pseudomonas aeruginosas* | 0,85 | > 3,44 |
| 32-31 | | *Staphylococcus aureus* | 0,73 | > 3,18 |
| 32-32 | Na⁺/Cu²⁺ (9/1) m= 100% | *Pseudomonas aeruginosas* | -0,06 | > 30,44 |
| 32-33 | | *Staphylococcus aureus* | 1,48 | > 3,18 |
| 32-34 | Ag⁺/Cu²⁺ (9/1) m= 100% | *Pseudomonas aeruginosas* | 2,23 | 3,44 |
| 32-35 | | *Staphylococcus aureus* | 3,18 | > 3,18 |
| 32-36 | Ag⁺/Cu²⁺ (1/9) m= 100% | *Pseudomonas aeruginosas* | 0,64 | > 3,44 |
| 32-37 | | *Staphylococcus aureus* | 3,18 | > 3,18 |
| 32-38 | Ag⁺/Fe²⁺ (1/1) m= 100% | *Pseudomonas aeruginosas* | > 3,52 | > 3,52 |
| 32-39 | | *Staphylococcus aureus* | 1,77 | > 3,54 |
| 32-40 | Ag+/Fe2+ (1/9) m= 100% | *Pseudomonas aeruginosas* | 0,52 | > 3,52 |
| 32-41 | | *Staphylococcus aureus* | 0,79 | > 3,54 |
| 32-42 | Ag⁺/Fe²⁺ (9/1) m= 100% | *Pseudomonas aeruginosas* | 2,52 | > 3,52 |
| 32-43 | | *Staphylococcus aureus* | 1,31 | > 3,54 |
| 32-44 | H⁺/Ag⁺ (1/9) m= 100% | *Pseudomonas aeruginosas* | > 3,52 | > 3,52 |
| 32-45 | | *Staphylococcus aureus* | 1,21 | > 3,54 |
| 32-46 | Ag⁺ réticulé à 26 % | *Pseudomonas aeruginosas* | 1,71 | > 3,52 |
| 32-47 | Ag⁺ réticulé à 26 % | *Staphylococcus aureus* | 1,03 | 3,54 |

| MELANGE DE POLYMERES | | | | |
|---|---|---|---|---|
| Polymere 1 | 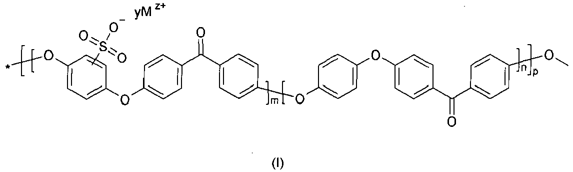 | | | |
| Polymere 2 | 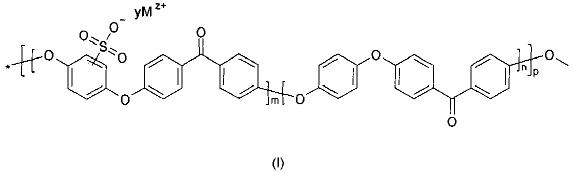 | | | |

| Exemples | Sel métallique M^{y+} | Microorganismes | Réduction logarithmique à t=0 | Réduction logarithmique à t=7 jours |
|---|---|---|---|---|
| 32-48 | M₁= Ag⁺ (1) | Pseudomonas aeruginosas | > 3,52 | > 3,52 |
| 32-49 | M₂= Fe²⁺ (9) m= 100% | Staphylococcus aureus | 0,68 | > 3,54 |

## Revendications

1. Nouveaux polymères de la famille des poly(aryl ether cétone)s ou poly(aryl ether sulfone)s contenant des fonctions sulfoniques ou sulfonates métalliques de formules I, II, III, IV, V, VI, VII, VIII, IX, X, XI, XII, XIII, XIV et XV 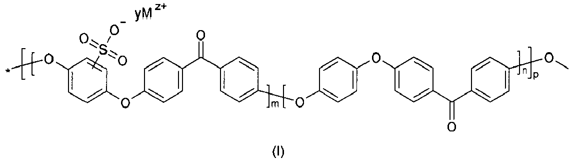 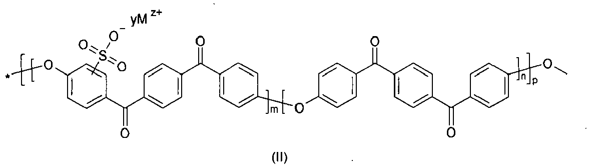 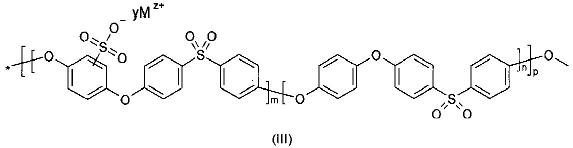 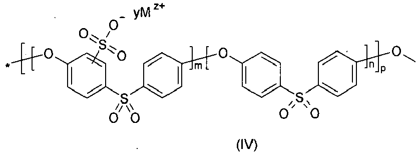 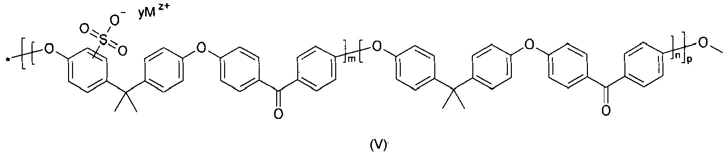 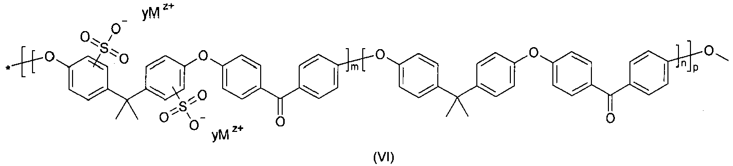 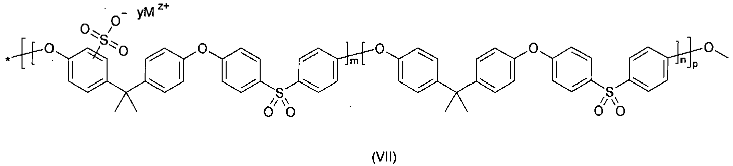 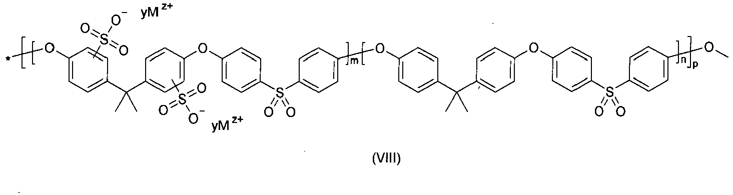 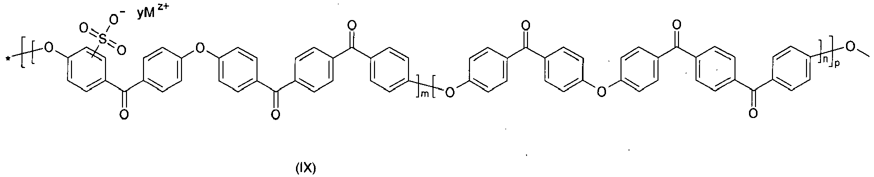 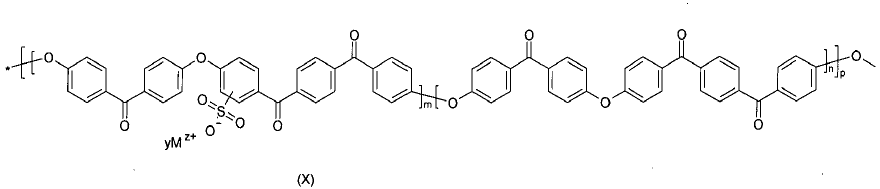 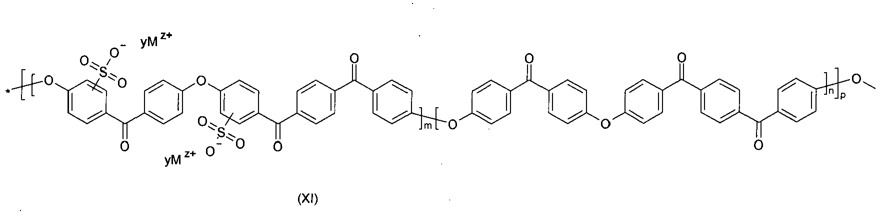 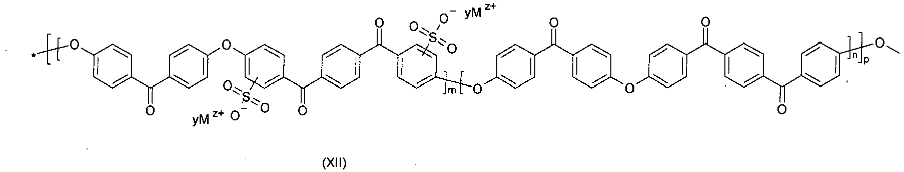 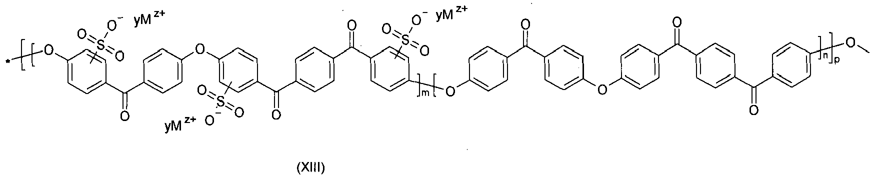 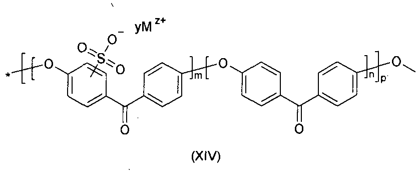 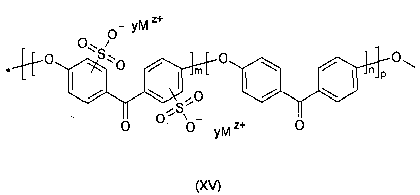 dans lesquelles :
- M^{Z+} représente un cation correspondant à une forme oxydée des éléments des colonnes IB, IIB, IIIA, IIIB, IVA, IVB, VA, VB, VIB, VIIB, VIIIB,
- z représente un entier de 1 à 6 correspondant au degré d'oxydation du métal,
- y représente un nombre entier ou fractionnaire égal à 1/z de manière à assurer la neutralité entre les charges négatives et positives,
- m représente le pourcentage d'unités polymériques ayant un motif oxoaryle ou dioxoaryle possédant une fonction sulfonate. Ce pourcentage varie entre 0,1 et 100%,
- n représente le pourcentage d'unités polymériques ayant aucun motif oxoaryle ou dioxoaryle fonctionnalisé par un motif sulfonate, n varie entre 0 et 99,9%,
- p représente le nombre d'unités polymériques du polymère ; p varie de 40 à 300.

2. Nouveaux polymères selon la revendication 1 de formules I, II, III, IV, V, VI, VII, VIII, IX, X, XI, XII, XIII, XIV et XV dans lesquelles :
- M^{Z+} représente un cation correspondant à une forme oxydée du titane, du zirconium, du vanadium, du chrome, du molybdène, du tungstène, du manganèse, du fer, du cuivre, de l'argent, de l'or, du zinc, de l'aluminium, du gallium, de l'indium, de l'étain, du bismuth,
- z représente un entier de 1 à 6 correspondant au degré d'oxydation du métal,
- y représente un nombre entier ou fractionnaire égal à 1/z de manière à assurer la neutralité entre les charges négatives et positives,
- m représente le pourcentage d'unités polymériques ayant un motif oxoaryle ou dioxoaryle possédant une fonction sulfonate, m varie entre 0,1 et 100%,
- n représente le pourcentage d'unités polymériques ayant aucun motif oxoaryle ou dioxoaryle fonctionnalisé par un motif sulfonate, n varie entre 0 et 99,9%,
- p représente le nombre d'unités polymériques du polymère, p varie de 40 à 300.

3. Nouveaux polymères selon la revendication 1 de formules I, II, III, IV, V, VI, VII, VIII, IX, X, XI, XII, XIII, XIV et XV dans lesquelles :
- M^{Z+} représente un cation correspondant à une forme oxydée du cuivre, de l'argent, de l'or, du zinc, du fer.
- z représente un entier de 1 à 6 correspondant au degré d'oxydation du métal,
- y représente un nombre entier ou fractionnaire égal à 1/z de manière à assurer la neutralité entre les charges négatives et positives,
- m représente le pourcentage d'unités polymériques ayant un motif oxoaryle ou dioxoaryle possédant une fonction sulfonate, m varie entre 0,1 et 100%,
- n représente le pourcentage d'unités polymériques ayant aucun motif oxoaryle ou dioxoaryle fonctionnalisé par un motif sulfonate, n varie entre 0 et 99,9%,
- p représente le nombre d'unités polymériques du polymère, p varie de 40 à 300.

4. Nouveaux polymères selon la revendication 1 de formules I, II, III, IV, V, VI, VII, VIII, IX, X, XI, XII, XIII, XIV et XV dans lesquelles :
- M^{Z+} représente un cation correspondant à une forme oxydée du cuivre, de l'argent, de l'or, du zinc, du fer.
- z représente un entier de 1 à 6 correspondant au degré d'oxydation du métal,
- y représente un nombre entier ou fractionnaire égal à 1/z de manière à assurer la neutralité entre les charges négatives et positives,
- m représente le pourcentage d'unités polymériques ayant un motif oxoaryle ou dioxoaryle possédant une fonction sulfonate, m varie entre 1 et 100%,
- n représente le pourcentage d'unités polymériques ayant aucun motif oxoaryle ou dioxoaryle fonctionnalisé par un motif sulfonate, n varie entre 0 et 99%.
- p représente le nombre d'unités polymériques du polymère, p varie de 60 et 200.

5. Procédé de synthèse des polymères selon les revendications 1 à 4 caractérisé que l'on réalise
1) la chlorosulfonation d'un polymère de formule XVI, XVII, XVIII, XIX, XX, XXI, XXII, XXIII par un mélange d'acide chlorosulfonique, de chlorure de thionyle et d'un amide selon un mode opératoire optimisé, 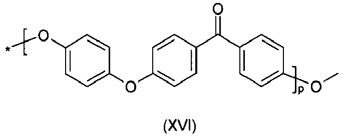 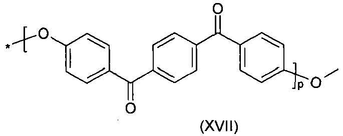 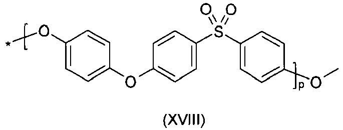 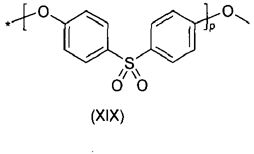 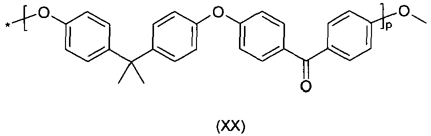 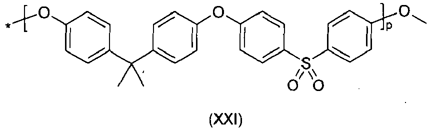 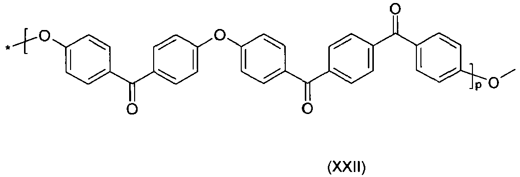 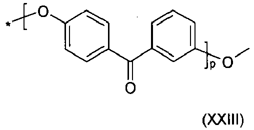 dans lesquelles
p représente le nombre d'unités polymériques du polymère, pp varie de 40 à 300,
pour obtenir les polymères de formule XXIV, XXV, XXVI, XVII, XXVIII, XIX, XXX, XXXI, XXXII, XXXIII, XXXIV, XXXV, XXXVI, XXXVII et XXXVIII 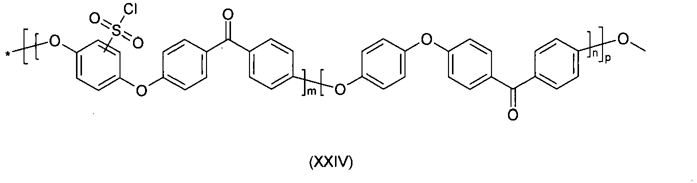 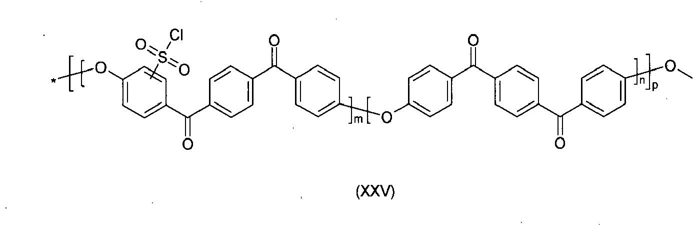 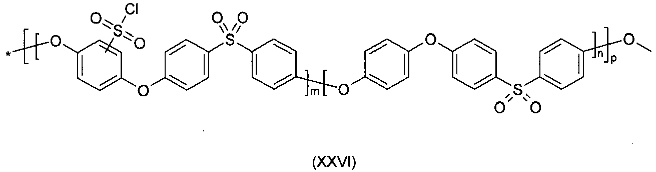 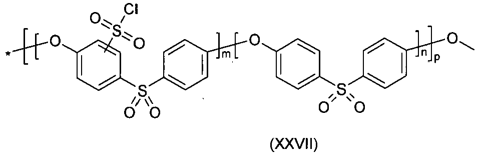 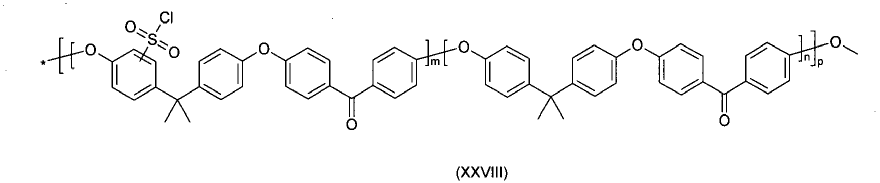 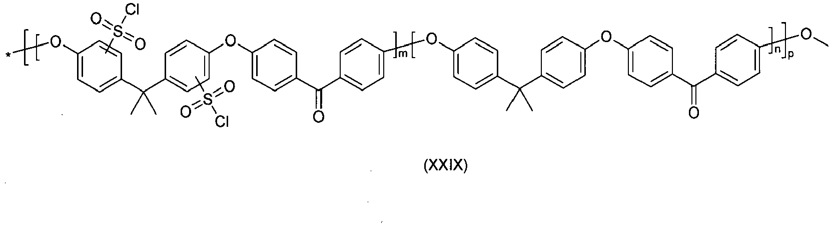 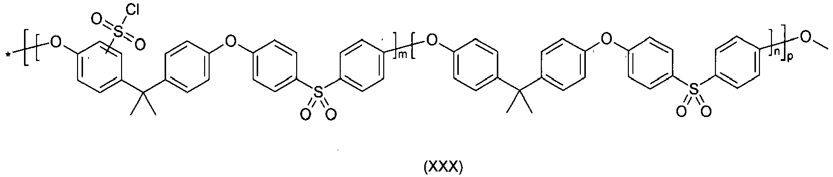 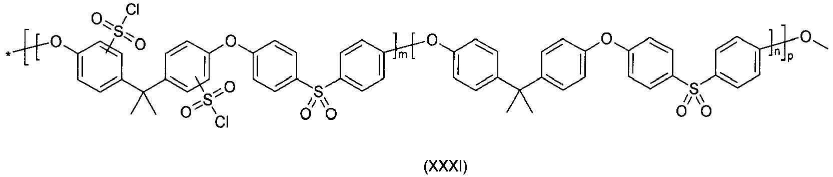 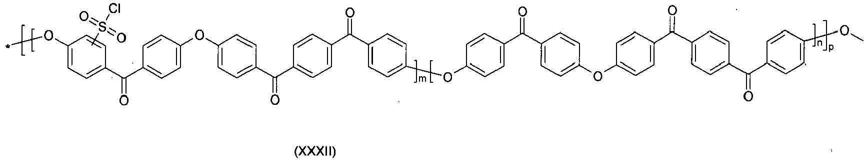 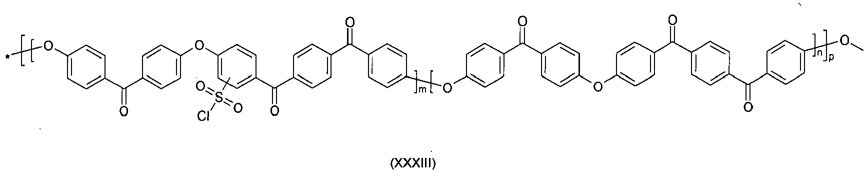 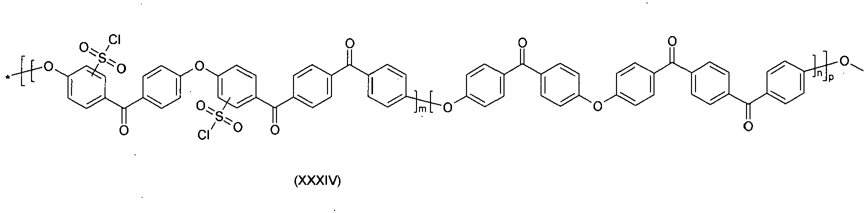 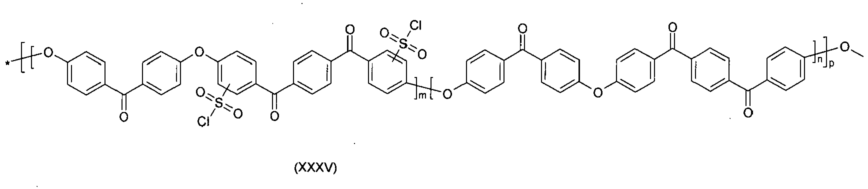 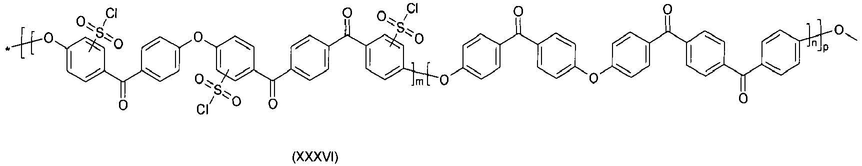 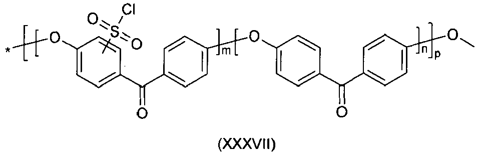 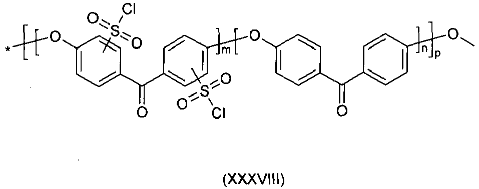 dans lesquelles :
- m représente le pourcentage d'unités polymériques ayant un motif oxoaryle ou dioxoaryle fonctionnalisé par un groupement chlorure de sulfonyle, m varie entre 0,1 et 100%,
- n représente le pourcentage d'unités polymériques ayant un motif dioxoaryle non fonctionnalisé par un groupement chlorure de sulfonyle, n varie entre 0 et 99,9%,
- p représente le nombre d'unités polymériques du polymère, p varie de 40 à 300,
2) ensuite, on fait réagir à une température comprises entre 60 et 140 °C, sur les polymères de formule XXIV, XXV, XXVI, XVII, XXVIII, XIX, XXX, XXXI, XXXII, XXXIII, XXXIV, XXXV, XXXVI, XXXVII et XXXVIII de l'eau pour obtenir les polymères de formule XIL, XL, XLI, XLII, XLIII, XLIV, XLV, XLVI, XLVII, XLVIII, XLIX, L, LI, LII, LIII, 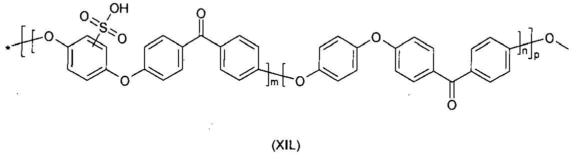 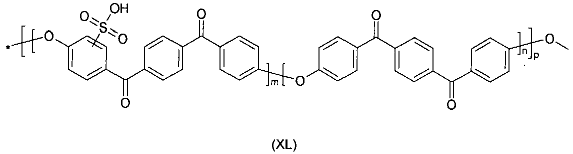 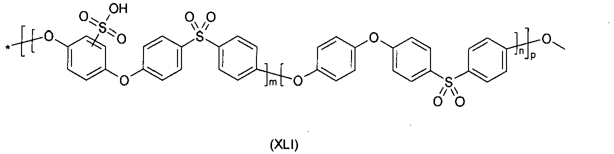 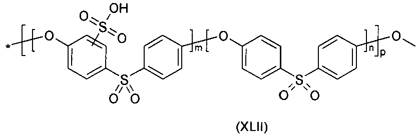 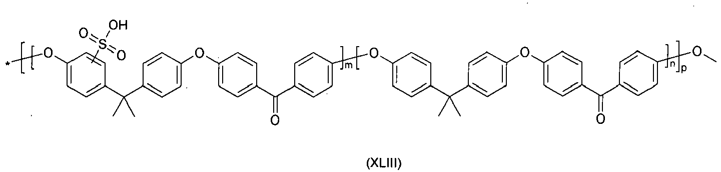 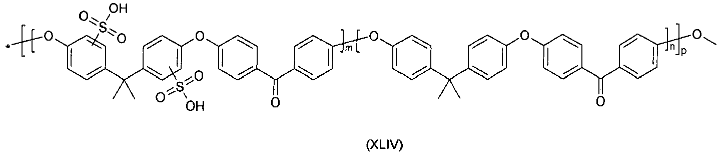 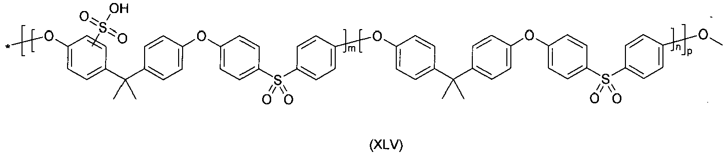 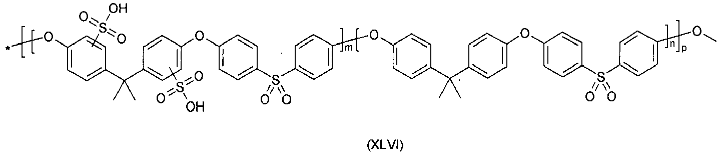 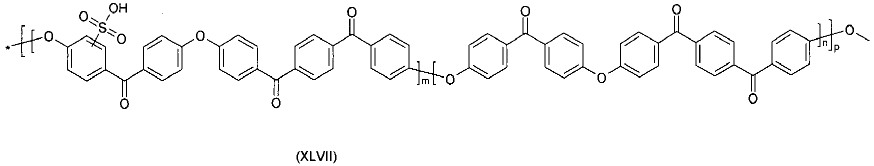 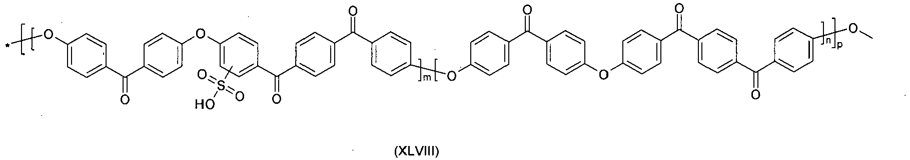 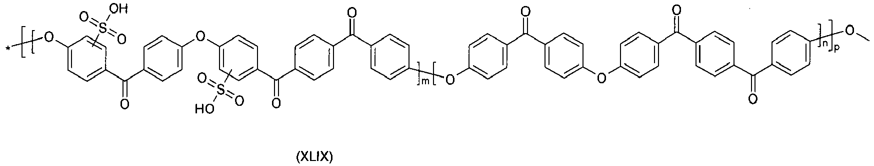 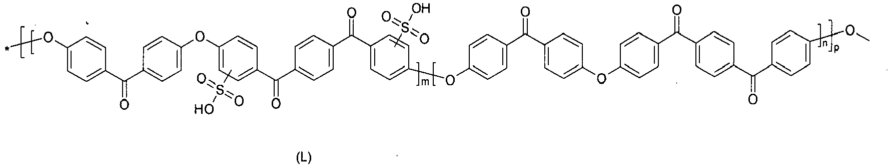 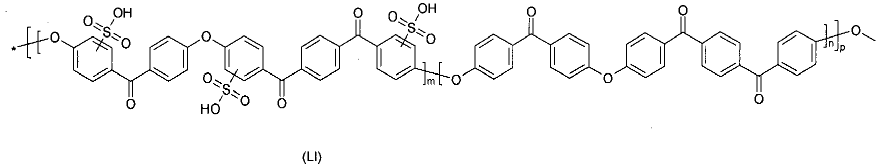 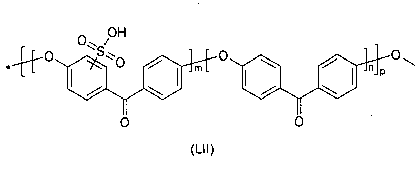 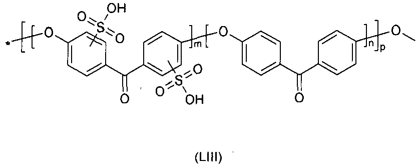 dans lesquelles :
- m représente le pourcentage d'unités polymériques ayant un motif oxoaryle ou dioxoaryle fonctionnalisé possédant une fonction sulfonique, m varie entre 0,1 et 100%,
- n représente le pourcentage d'unités polymériques ayant un motif oxoaryle ou dioxoaryle non fonctionnalisé par une fonction sulfonique, n varie entre 0 et 99,9%,
- p représente le nombre d'unités polymériques du polymère, p varie de 40 à 300,
3) et ensuite, on fait réagir les polymères de formule XIL, XL, XLI, XLII, XLIII, XLIV, XLV, XLVI, XLVII, XLVIII, XLIX, L, LI, LII et LIII avec le sel métallique du métal M ou directement le métal M au degré d'oxydation 0 en présence d'ultrason.

6. Procédé de synthèse selon la revendication 5 **caractérisé en ce que**
1) premièrement, la chlorosulfonation d'un polymère de formule XVI, XVII, XVIII, XIX, XX, XXI, XXII, XXIII par un mélange d'acide chlorosulfonique, de chlorure de thionyle et d'un amide selon un mode opératoire optimisé, dans lesquelles
p représente le nombre d'unités polymériques du polymère, p varie de 60 et 200,
pour obtenir les polymères de formules XXIV, XXV, XXVI, XVII, XXVIII, XIX, XXX, XXXI, XXXII, XXXIII, XXXIV, XXXV, XXXVI, XXXVII et XXXVIII dans lesquelles :
- m représente le pourcentage d'unités polymériques ayant un motif oxoaryle ou dioxoaryle fonctionnalisé par un groupement chlorosulfoné, m varie entre 1 et 100%.
- n représente le pourcentage d'unités polymériques ayant un motif oxoaryle ou dioxoaryle non fonctionnalisé par un groupement chlorosulfoné, n varie entre 0 et et 99% ;
- p représente le nombre d'unités polymériques du polymère, p varie de 60 à 200,
2) deuxièmement, on fait réagir à une température comprises entre 90 et 120 °C sur les polymères de formule XXIV, XXV, XXVI, XVII, XXVIII, XIX, XXX, XXXI, XXXII, XXXIII, XXXIV, XXXV, XXXVI, XXXVII et XXXVIII de l'eau pour obtenir les polymères de formules XIL, XL, XLI, XLII, XLIII, XLIV, XLV, XLVI, XLVII, XLVIII, XLIX, L, LI, LII, LIII, dans lesquelles :
- m représente le pourcentage d'unités polymériques ayant un motif oxoaryle ou dioxoaryle fonctionnalisé possédant une fonction sulfonique. Ce pourcentage varie entre 1 et 100%,
- n représente le pourcentage d'unités polymériques ayant un motif oxoaryle ou dioxoaryle non fonctionnalisé par une fonction sulfonique. Ce pourcentage varie entre 0 et 99%
- p représente le nombre d'unités polymériques du polymère; p varie de 60 et 200,
3) et finalement, on fait réagir dans une première variante les polymères de formule XIL, XL, XLI, XLII, XLIII, XLIV, XLV, XLVI, XLVII, XLVIII, XLIX, L, LI, LII et LIII avec le sel métallique du métal M ou directement le métal M au au degré d'oxydation 0 en présence d'ultrason.

7. Procédé selon les revendications 5 et 6, **caractérisé en ce que** la chlorosulfonation est réalisée à une température comprise entre 0 et 80 °C par un mélange de 0,1 à 5 équivalents d'acide chlorosulfonique, 0,1 à 15 équivalents de chlorure de thionyle en présence de 0,1 à 5 équivalents d'un amide par rapport aux motifs oxoaryles ou dioxoaryles à chlorosulfoner pour obtenir des taux de chlorosulfonation compris entre 0,1 et 50%.

8. Procédé selon la revendication 5 et 6, **caractérisé en ce que** les sels métalliques utilisés pour la réaction avec les polymères de formules XIL, XL, XLI, XLII, XLIII, XLIV, XLV, XLVI, XLVII, XLVIII, XLIX, L, LI, LII et LIII sont des chlorures, des bromures, des carbonates, des alkyl carboxylates, des acétates, des propionates, des arylcarboxylates, des benzoates, des acétylacétates, des acétylacétonates, des alcoolates.

9. Procédé selon la revendication 5 et 6, **caractérisé en ce que** les sels métalliques préférés sont des sels d'acides faibles de pKa dont le pKa est supérieur à 0 comme les carbonates, les acétates, les benzoates, les alcoolates, les acétylacétates, les acétylacétonates

10. Procédé selon la revendication 5 et 6, **caractérisé en ce que** l'on fait réagir avec les polymères de formule XIL, XL, XLI, XLII, XLIII, XLIV, XLV, XLVI, XLVII, XLVIII, XLIX, L, LI, LII et LIII avec un métal au degré d'oxydation 0 comme par exemple l'indium, le fer, le cuivre sous ultrason.

11. Les polymères selon les revendications 1 à 4 de formules I, II, III, IV, V, VI, VII, VIII, IX, X, XI, XII, XIII, XIV, XV présentent une activité contre les bacteries, les champignons, les microbes.

12. Les polymères selon les revendications 1 à 4 de formules I, II, III, IV, V, VI, VII, VIII, IX, X, XI, XII, XIII, XIV, XV présentent une activité contre les bacteries.

13. Les polymères selon les revendications 1 à 4 de formules I, II, III, IV, V, VI, VII, VIII, IX, X, XI, XII, XIII, XIV, XV présentent une activité contre les bacteries comme Staphilococcaus Aureus et Pseudomonas aeruginosa.

14. Les polymères selon les revendications 1 à 4 de formules I, II, III, IV, V, VI, VII, VIII, IX, X, XI, XII, XIII, XIV, XV présentent une activité contre les champignons.

15. Les polymères selon les revendications 1 à 4 de formules I, II, III, IV, V, VI, VII, VIII, IX, X, XI, XII, XIII, XIV, XV présentent une activité contre les microbes.

16. Les polymères selon la revendication 5 de formule XIL, XL, XLI, XLII, XLIII, XLIV, XLV, XLVI, XLVII, XLVIII, XLIX, L, LI, LII, LIII présente une activité contre les microbes, les champignons et les bacteries.

17. Les polymères selon la revendication 5 de formule XIL, XL, XLI, XLII, XLIII, XLIV, XLV, XLVI, XLVII, XLVIII, XLIX, L, LI, LII, LIII présente une activité contre les champignons.

18. Les polymères selon la revendication 5 de formule XIL, XL, XLI, XLII, XLIII, XLIV, XLV, XLVI, XLVII, XLVIII, XLIX, L, LI, LII, LIII présente une activité contre les microbes.

19. Les polymères selon la revendication 5 de formule XIL, XL, XLI, XLII, XLIII, XLIV, XLV, XLVI, XLVII, XLVIII, XLIX, L, LI, LII, LIII présente une activité contre les bacteries.

## Patentansprüche

1. Neue Polymere der Familie der Poly(aryletherketon)e oder Poly(arylethersulfon)e, die Sulfonsäure- oder Metallsulfonat-Funktionen der Formeln I, II, III, IV, V, VI, VII, VIII, IX, X, XI, XII, XIII, XIV und XV enthalten 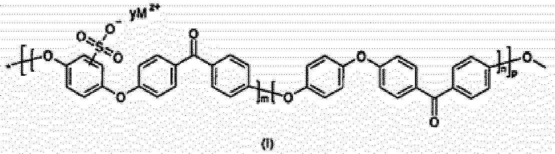 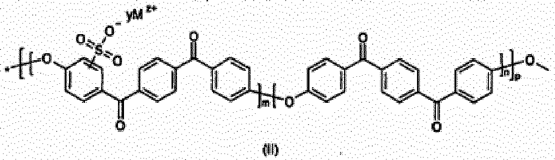 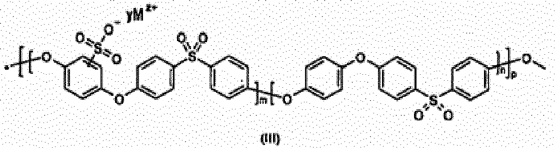 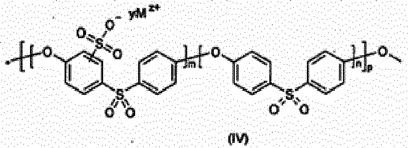 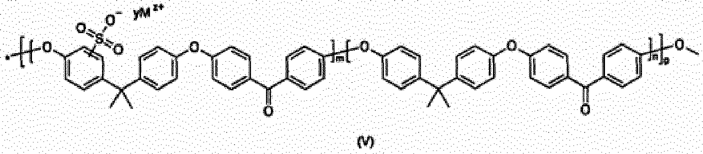 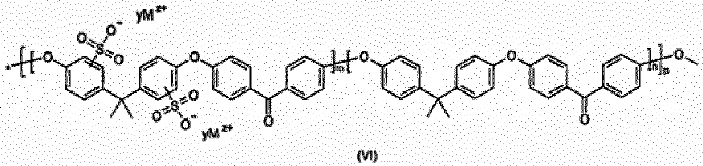 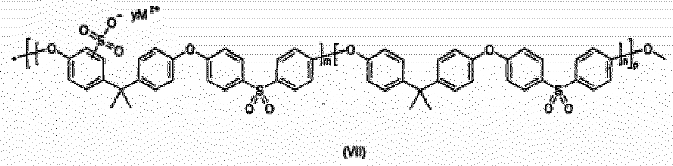 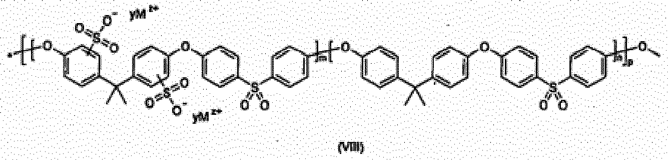 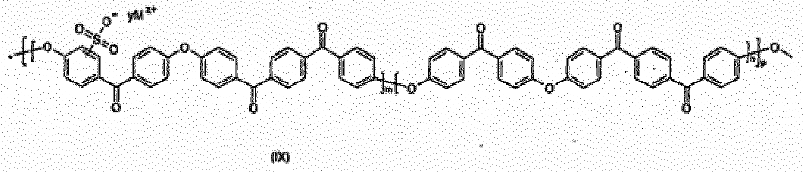 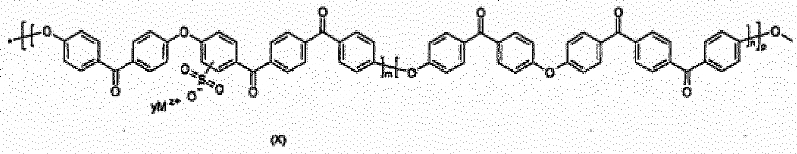 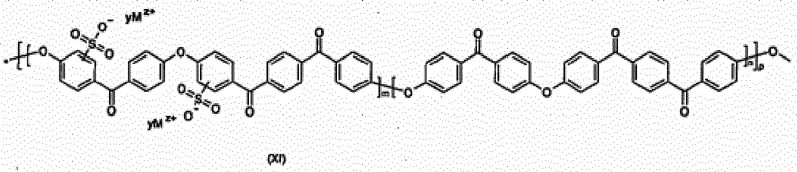 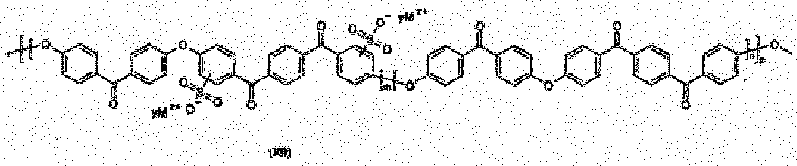 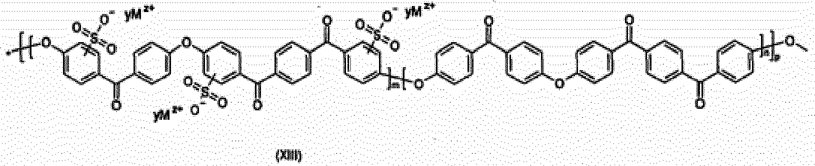 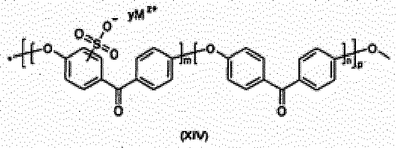 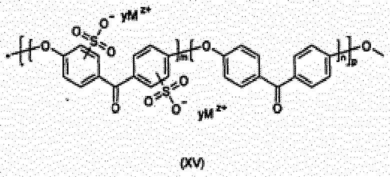 wobei:
- M^{Z+} für ein Kation steht, das einer oxidierten Form der Elemente der Spalten I B, II B, III A, III B, IV A, IV B, V A, V B, VI B, VII B, VIII B entspricht,
- z für eine ganze Zahl von 1 bis 6 steht, die dem Oxidationsgrad des Metalls entspricht,
- y für eine ganze oder Bruchzahl von gleich 1/z steht, sodass die Neutralität zwischen den negativen und positiven Ladungen sichergestellt wird,
- m für den Prozentanteil an Polymereinheiten steht, die ein Oxoaryl- oder Dioxoarylmuster aufweisen, welches eine Sulfonatfunktion besitzt. Dieser Prozentanteil variiert zwischen 0,1 und 100 %,
- n für den Prozentanteil an Polymereinheiten steht, die kein durch ein Sulfonatmuster funktionalisiertes Oxoaryl- oder Dioxoarylmuster aufweisen, n zwischen 0 und 99,9 % variiert,
- p für die Anzahl an Polymereinheiten des Polymers steht; p von 40 bis 300 variiert.

2. Neue Polymere nach Anspruch 1 der Formeln I, II, III, IV, V, VI, VII, VIII, IX, X, XI, XII, XIII, XIV und XV, wobei:
- M^{z+} für ein Kation steht, das einer oxidierten Form von Titan, Zirkonium, Vanadium, Chrom, Molybdän, Wolfram, Mangan, Eisen, Kupfer, Silber, Gold, Zink, Aluminium, Gallium, Indium, Zinn, Bismut entspricht,
- z für eine ganze Zahl von 1 bis 6 steht, die dem Oxidationsgrad des Metalls entspricht,
- y für eine ganze oder Bruchzahl von gleich 1/z steht, sodass die Neutralität zwischen den negativen und positiven Ladungen sichergestellt wird,
- m für den Prozentanteil an Polymereinheiten steht, die ein Oxoaryl- oder Dioxoarylmuster aufweisen, welches eine Sulfonatfunktion besitzt, m zwischen 0,1 und 100 % variiert,
- n für den Prozentanteil an Polymereinheiten steht, die kein durch ein Sulfonatmuster funktionalisiertes Oxoaryl- oder Dioxoarylmuster aufweisen, n zwischen 0 und 99,9 % variiert,
- p für die Anzahl an Polymereinheiten des Polymers steht, p von 40 bis 300 variiert.

3. Neue Polymere nach Anspruch 1 der Formeln I, II, III, IV, V, VI, VII, VIII, IX, X, XI, XII, XIII, XIV und XV, wobei:
- M^{Z+} für ein Kation steht, das einer oxidierten Form von Kupfer, Silber, Gold, Zink, Eisen entspricht,
- z für eine ganze Zahl von 1 bis 6 steht, die dem Oxidationsgrad des Metalls entspricht,
- y für eine ganze oder Bruchzahl von gleich 1/z steht, sodass die Neutralität zwischen den negativen und positiven Ladungen sichergestellt wird,
- m für den Prozentanteil an Polymereinheiten steht, die ein Oxoaryl- oder Dioxoarylmuster aufweisen, welches eine Sulfonatfunktion besitzt, m zwischen 0,1 und 100 % variiert,
- n für den Prozentanteil an Polymereinheiten steht, die kein durch ein Sulfonatmuster funktionalisiertes Oxoaryl- oder Dioxoarylmuster aufweisen, n zwischen 0 und 99,9 % variiert,
- p für die Anzahl an Polymereinheiten des Polymers steht, p von 40 bis 300 variiert.

4. Neue Polymere nach Anspruch 1 der Formeln I, II, III, IV, V, VI, VII, VIII, IX, X, XI, XII, XIII, XIV und XV, wobei:
- M^{Z+} für ein Kation steht, das einer oxidierten Form von Kupfer, Silber, Gold, Zink, Eisen entspricht,
- z für eine ganze Zahl von 1 bis 6 steht, die dem Oxidationsgrad des Metalls entspricht,
- y für eine ganze oder Bruchzahl von gleich 1/z steht, sodass die Neutralität zwischen den negativen und positiven Ladungen sichergestellt wird,
- m für den Prozentanteil an Polymereinheiten steht, die ein Oxoaryl- oder Dioxoarylmuster aufweisen, welches eine Sulfonatfunktion besitzt, m zwischen 1 und 100 % variiert,
- n für den Prozentanteil an Polymereinheiten steht, die kein durch ein Sulfonatmuster funktionalisiertes Oxoaryl- oder Dioxoarylmuster aufweisen, n zwischen 0 und 99 % variiert,
- p für die Anzahl an Polymereinheiten des Polymers steht, p von 60 und 200 variiert.

5. Verfahren zur Synthese der Polymere nach den Ansprüchen 1 bis 4, **dadurch gekennzeichnet, dass** ausgeführt wird
1) die Chlorsulfonierung eines Polymers der Formel XVI, XVII, XVIII, XIX, XX, XXI, XXII, XXIII durch eine Mischung aus Chlorsulfonsäure, Thionylchlorid und einem Amid nach einer optimierten Vorgehensweise, 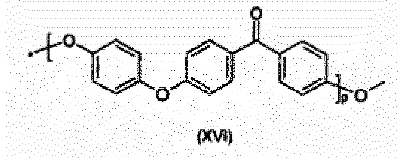 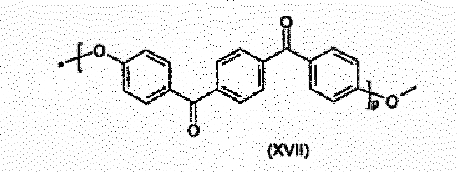 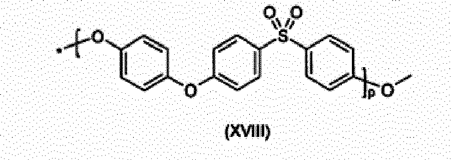 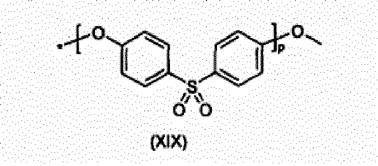 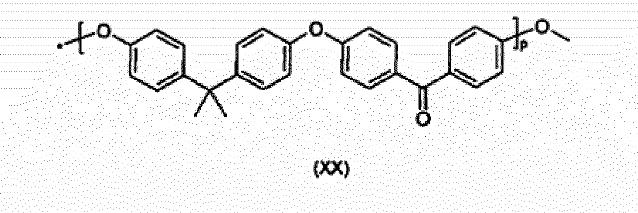 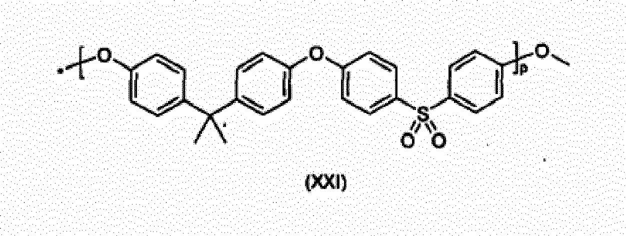 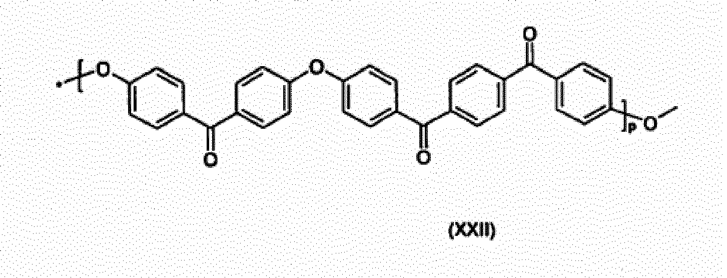 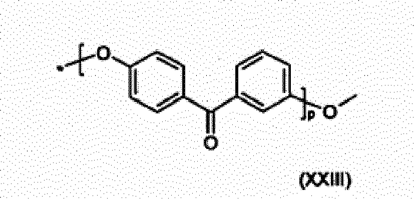 wobei
p für die Anzahl an Polymereinheiten des Polymers steht, pp von 40 bis 300 variiert,
um die Polymere der Formel XXIV, XXV, XXVI, XVII, XXVIII, XIX, XXX, XXXI, XXXII, XXXIII, XXXIV, XXXV, XXXVI, XXXVII und XXXVIII zu erhalten 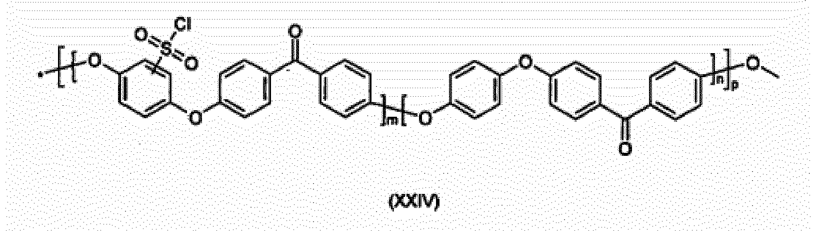 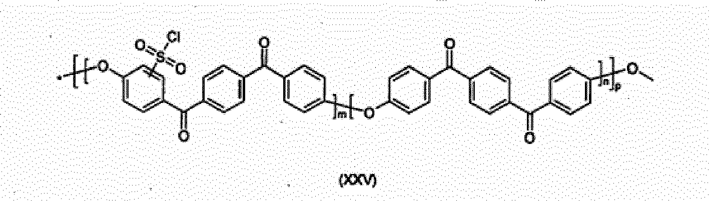 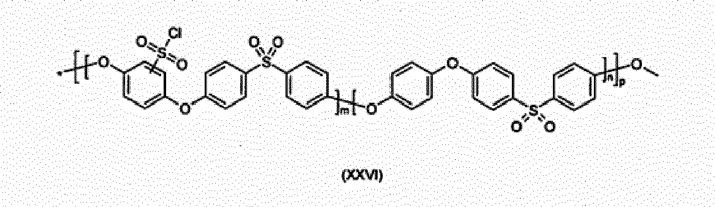 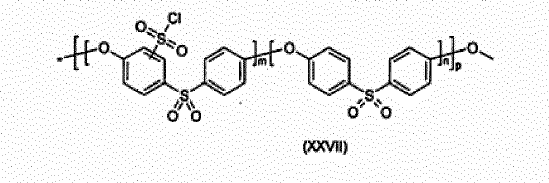 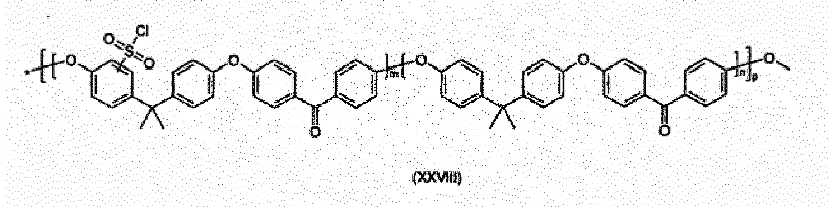 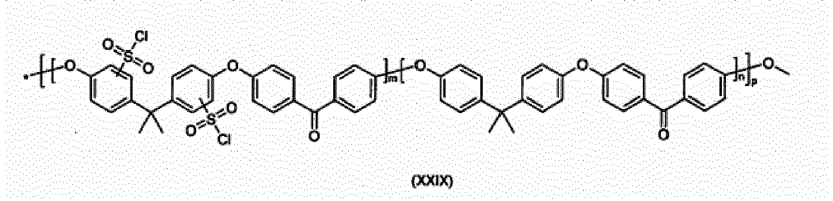 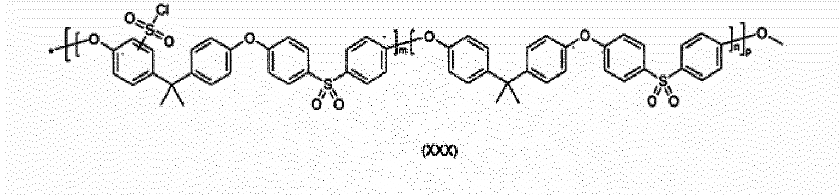 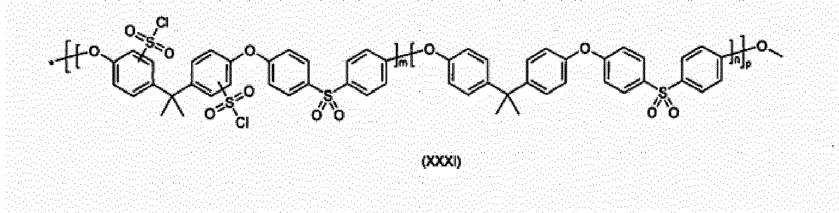 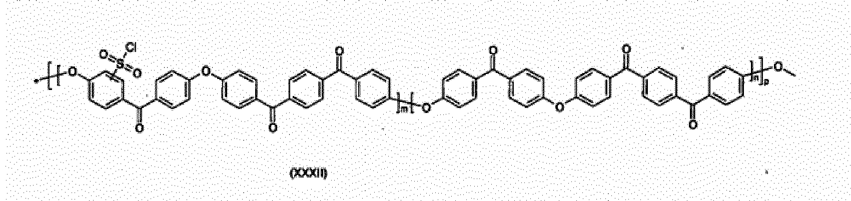 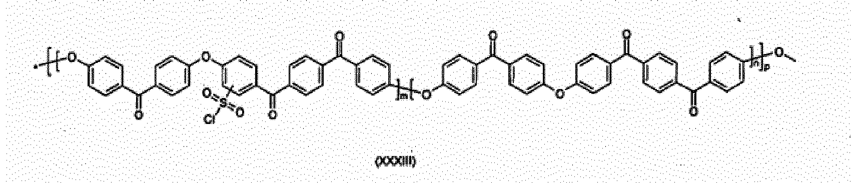 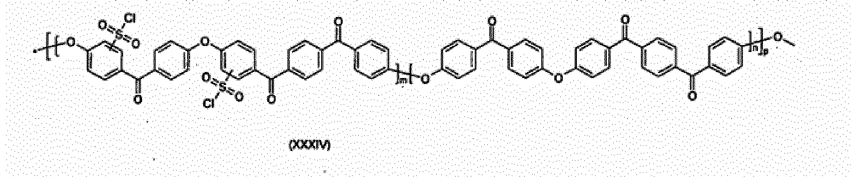 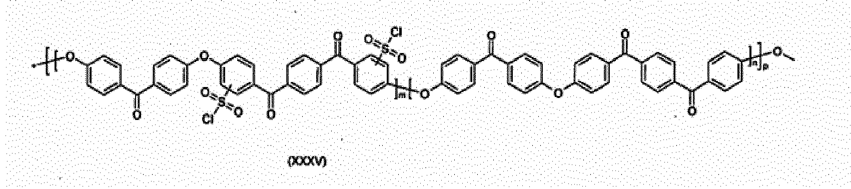 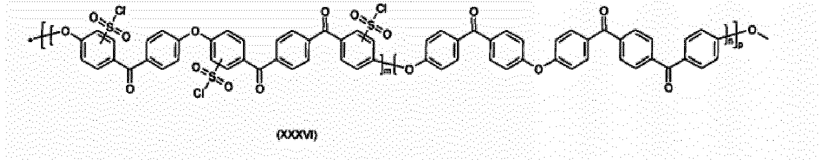 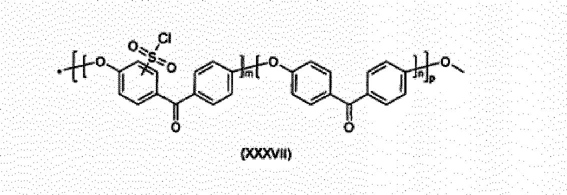 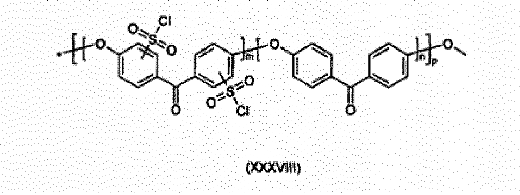 wobei:
- m für den Prozentanteil an Polymereinheiten steht, die ein Oxoaryl- oder Dioxoarylmuster aufweisen, welches durch eine Sulfonylchloridgruppe funktionalisiert ist, m zwischen 0,1 und 100 % variiert,
- n für den Prozentanteil an Polymereinheiten steht, die ein Dioxoarylmuster aufweisen, welches nicht durch eine Sulfonylchloridgruppe funktionalisiert ist, n zwischen 0 und 99,9 % variiert,
- p für die Anzahl an Polymereinheiten des Polymers steht, p von 40 bis 300 variiert,
2) anschließend bei einer Temperatur im Bereich zwischen 60 und 140 °C Wasser an den Polymeren der Formel XXIV, XXV, XXVI, XVII, XXVIII, XIX, XXX, XXXI, XXXII, XXXIII, XXXIV, XXXV, XXXVI, XXXVII und XXXVIII reagiert wird, um die Polymere der Formel XIL, XL, XLI, XLII, XLIII, XLIV, XLV, XLVI, XLVII, XLVIII, XLIX, L, LI, LII, LIII zu erhalten, 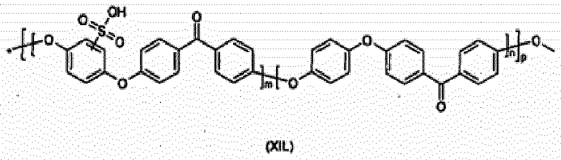 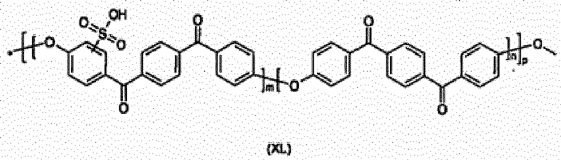 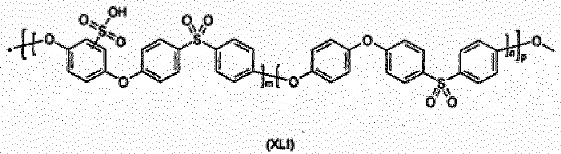 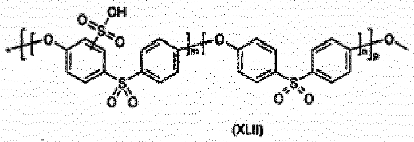 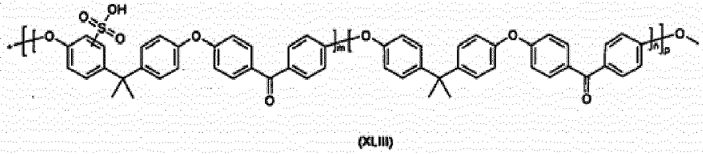 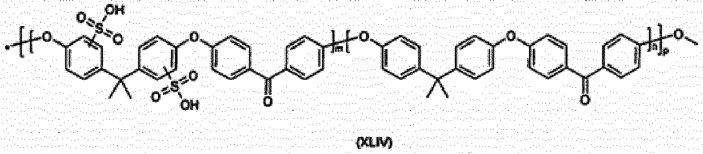 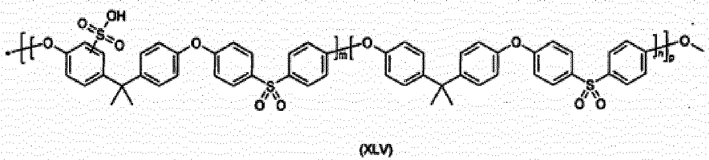 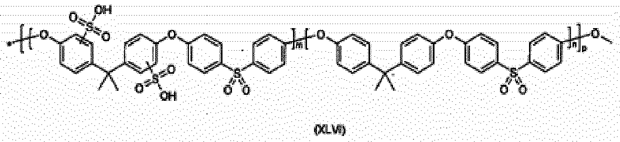 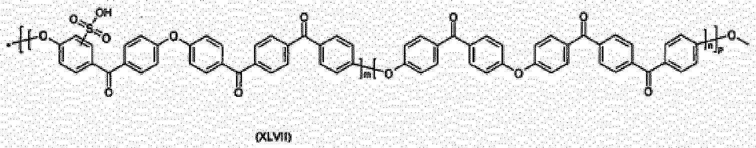 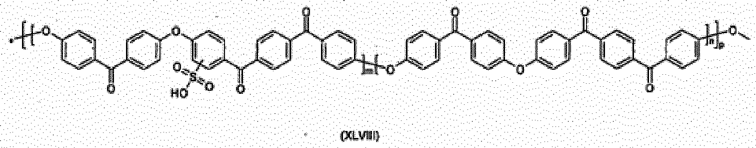 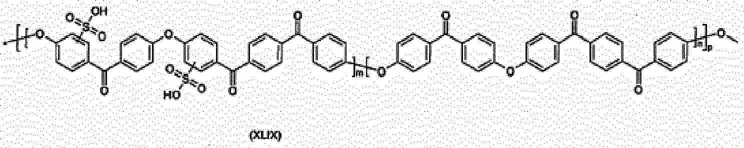 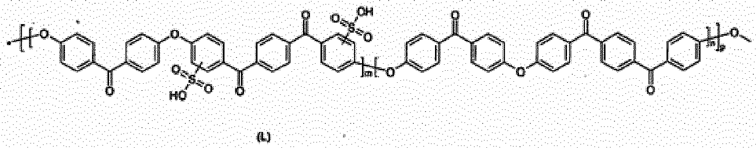 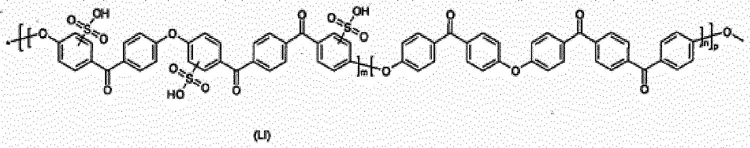 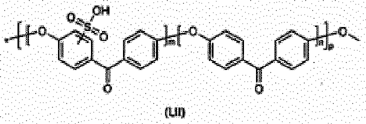 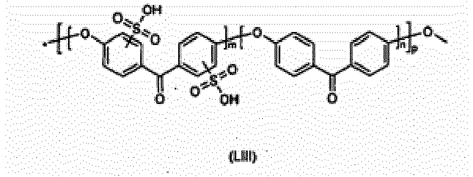 wobei:
- m für den Prozentanteil an Polymereinheiten steht, die ein funktionalisiertes Oxoaryl- oder Dioxoarylmuster aufweisen, welches eine Sulfonsäurefunktion besitzt, m zwischen 0,1 und 100 % variiert,
- n für den Prozentanteil an Polymereinheiten steht, die ein nicht durch eine Sulfonsäurefunktion funktionalisiertes Oxoaryl- oder Dioxoarylmuster aufweisen, n zwischen 0 und 99,9 % variiert,
- p für die Anzahl an Polymereinheiten des Polymers steht, p von 40 bis 300 variiert,
3) und anschließend die Polymere der Formel XIL, XL, XLI, XLII, XLIII, XLIV, XLV, XLVI, XLVII, XLVIII, XLIX, L, LI, LII und LIII mit dem Metallsalz des Metalls M oder direkt dem Metall M auf dem Oxidationsgrad 0 in Gegenwart von Ultraschall reagiert werden.

6. Syntheseverfahren nach Anspruch 5, **dadurch gekennzeichnet, dass**
1) erstens die Chlorsulfonierung eines Polymers der Formel XVI, XVII, XVIII, XIX, XX, XXI, XXII, XXIII durch eine Mischung aus Chlorsulfonsäure, Thionylchlorid und einem Amid nach einer optimierten Vorgehensweise, wobei
p für die Anzahl an Polymereinheiten des Polymers steht, p von 60 und 200 variiert,
um die Polymere der Formeln XXIV, XXV, XXVI, XVII, XXVIII, XIX, XXX, XXXI, XXXII, XXXIII, XXXIV, XXXV, XXXVI, XXXVII und XXXVIII zu erhalten, wobei:
- m für den Prozentanteil an Polymereinheiten steht, die ein durch eine chlorsulfonierte Gruppe funktionalisiertes Oxoaryl- oder Dioxoarylmuster aufweisen, m zwischen 1 und 100 % variiert,
- n für den Prozentanteil an Polymereinheiten steht, die ein nicht durch eine chlorsulfonierte Gruppe funktionalisiertes Oxoaryl- oder Dioxoarylmuster aufweisen, n zwischen 0 und und 99 % variiert;
- p für die Anzahl an Polymereinheiten des Polymers steht, p von 60 bis 200 variiert,
2) zweitens bei einer Temperatur im Bereich zwischen 90 und 120 °C Wasser an den Polymeren der Formel XXIV, XXV, XXVI, XVII, XXVIII, XIX, XXX, XXXI, XXXII, XXXIII, XXXIV, XXXV, XXXVI, XXXVII und XXXVIII reagiert wird, um die Polymere der Formeln XIL, XL, XLI, XLII, XLIII, XLIV, XLV, XLVI, XLVII, XLVIII, XLIX, L, LI, LII, LIII zu erhalten, wobei:
- m für den Prozentanteil an Polymereinheiten steht, die ein funktionalisiertes Oxoaryl- oder Dioxoarylmuster aufweisen, welches eine Sulfonsäurefunktion besitzt. Dieser Prozentanteil variiert zwischen 1 und 100 %,
- n für den Prozentanteil an Polymereinheiten steht, die ein nicht durch eine Sulfonsäurefunktion funktionalisiertes Oxoaryl- oder Dioxoarylmuster aufweisen. Dieser Prozentanteil variiert zwischen 0 und 99 %,
- p für die Anzahl an Polymereinheiten des Polymers steht; p von 60 und 200 variiert,
3) und schließlich bei einer ersten Variante die Polymere der Formel XIL, XL, XLI, XLII, XLIII, XLIV, XLV, XLVI, XLVII, XLVIII, XLIX, L, LI, LII und LIII mit dem Metallsalz des Metalls M oder direkt dem Metall M auf dem auf dem Oxidationsgrad 0 in Gegenwart von Ultraschall reagiert werden.

7. Verfahren nach den Ansprüchen 5 und 6, **dadurch gekennzeichnet, dass** die Chlorsulfonierung bei einer Temperatur im Bereich zwischen 0 und 80 °C durch eine Mischung aus 0,1 bis 5 Äquivalenten Chlorsulfonsäure, 0,1 bis 15 Äquivalenten Thionylchlorid in Gegenwart von 0,1 bis 5 Äquivalenten eines Amids bezogen auf die Oxoaryl- oder Dioxoarylmuster, die chlorsulfoniert werden sollen, ausgeführt wird, um Chlorsulfonierungsraten im Bereich zwischen 0,1 und 50 % zu erhalten.

8. Verfahren nach Anspruch 5 und 6, **dadurch gekennzeichnet, dass** die Metallsalze, die für die Reaktion mit den Polymeren der Formeln XIL, XL, XLI, XLII, XLIII, XLIV, XLV, XLVI, XLVII, XLVIII, XLIX, L, LI, LII und LIII verwendet werden, Chloride, Bromide, Carbonate, Alkylcarboxylate, Acetate, Propionate, Arylcarboxylate, Benzoate, Acetylacetate, Acetylacetonate, Alkoholate sind.

9. Verfahren nach Anspruch 5 und 6, **dadurch gekennzeichnet, dass** die bevorzugten Metallsalze Salze von Säuren mit schwacher pKS sind, deren pKS größer als 0 ist, wie Carbonate, Acetate, Benzoate, Alkoholate, Acetylacetate, Acetylacetonate.

10. Verfahren nach Anspruch 5 und 6, **dadurch gekennzeichnet, dass** mit den Polymeren der Formel XIL, XL, XLI, XLII, XLIII, XLIV, XLV, XLVI, XLVII, XLVIII, XLIX, L, LI, LII und LIII mit einem Metall auf dem Oxidationsgrad 0, wie zum Beispiel Indium, Eisen, Kupfer, unter Ultraschall reagiert wird.

11. Polymere nach den Ansprüchen 1 bis 4 der Formeln I, II, III, IV, V, VI, VII, VIII, IX, X, XI, XII, XIII, XIV, XV, die eine Aktivität gegen Bakterien, Pilze, Mikroben aufweisen.

12. Polymere nach den Ansprüchen 1 bis 4 der Formeln I, II, III, IV, V, VI, VII, VIII, IX, X, XI, XII, XIII, XIV, XV, die eine Aktivität gegen Bakterien aufweisen.

13. Polymere nach den Ansprüchen 1 bis 4 der Formeln I, II, III, IV, V, VI, VII, VIII, IX, X, XI, XII, XIII, XIV, XV, die eine Aktivität gegen Bakterien wie Staphylococcus Aureus und Pseudomonas aeruginosa aufweisen.

14. Polymere nach den Ansprüchen 1 bis 4 der Formeln I, II, III, IV, V, VI, VII, VIII, IX, X, XI, XII, XIII, XIV, XV, die eine Aktivität gegen Pilze aufweisen.

15. Polymere nach den Ansprüchen 1 bis 4 der Formeln I, II, III, IV, V, VI, VII, VIII, IX, X, XI, XII, XIII, XIV, XV, die eine Aktivität gegen Mikroben aufweisen.

16. Polymere nach Anspruch 5 der Formel XIL, XL, XLI, XLII, XLIII, XLIV, XLV, XLVI, XLVII, XLVIII, XLIX, L, LI, LII, LIII, die eine Aktivität gegen Mikroben, Pilze und Bakterien aufweisen.

17. Polymere nach Anspruch 5 der Formel XIL, XL, XLI, XLII, XLIII, XLIV, XLV, XLVI, XLVII, XLVIII, XLIX, L, LI, LII, LIII, die eine Aktivität gegen Pilze aufweisen.

18. Polymere nach Anspruch 5 der Formel XIL, XL, XLI, XLII, XLIII, XLIV, XLV, XLVI, XLVII, XLVIII, XLIX, L, LI, LII, LIII, die eine Aktivität gegen Mikroben aufweisen.

19. Polymere nach Anspruch 5 der Formel XIL, XL, XLI, XLII, XLIII, XLIV, XLV, XLVI, XLVII, XLVIII, XLIX, L, LI, LII, LIII, die eine Aktivität gegen Bakterien aufweisen.

## Claims

1. Novel polymers of the family of poly(aryl ether ketone)s or poly(aryl ether sulfone)s containing sulfonate or metal sulfonate functions of formulas I, II, III, IV, V, VI, VII, VIII, IX, X, XI, XII, XIII, XIV and XV 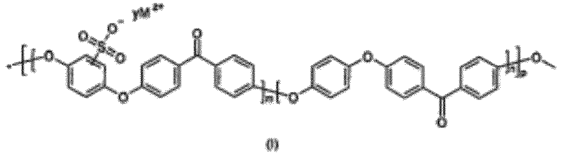 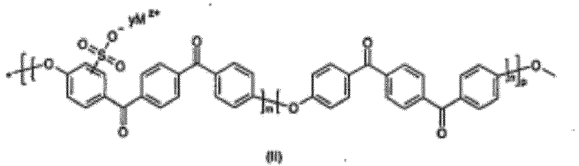 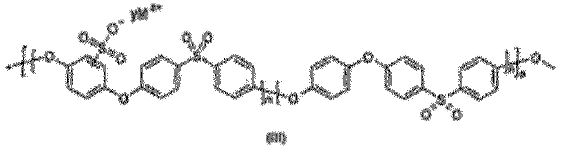 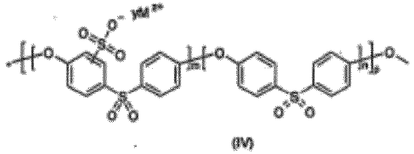 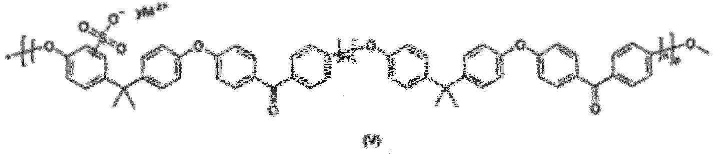 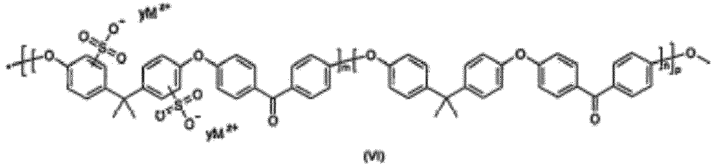 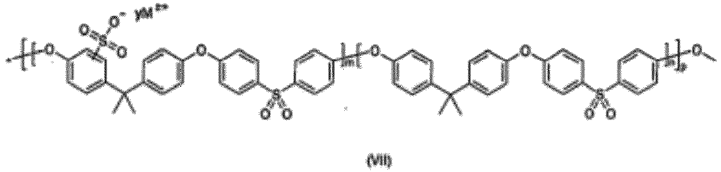 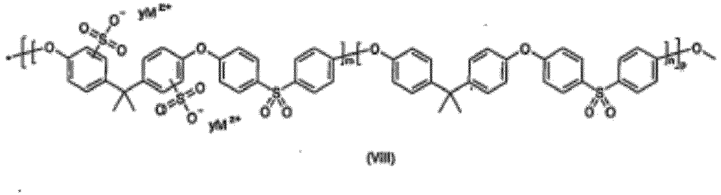 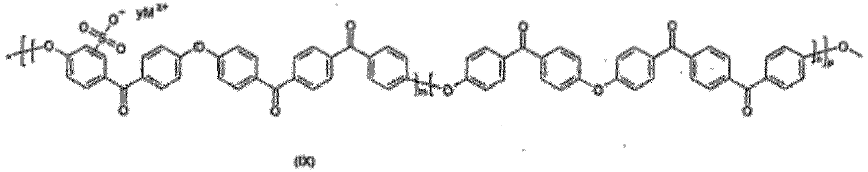 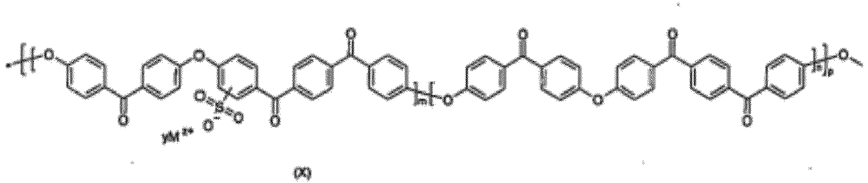 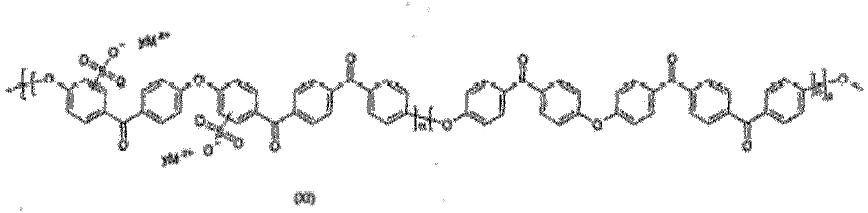 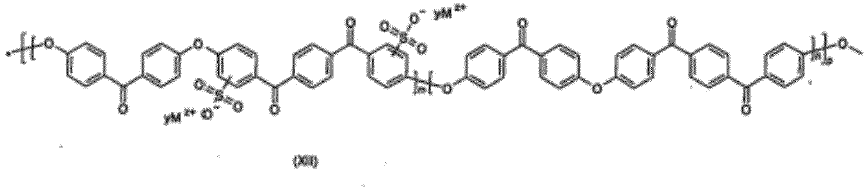 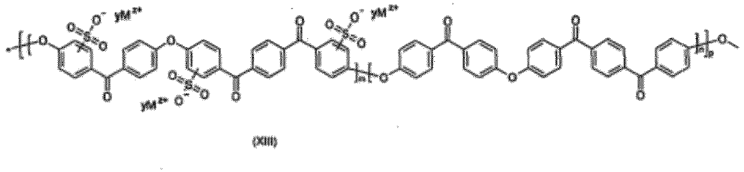 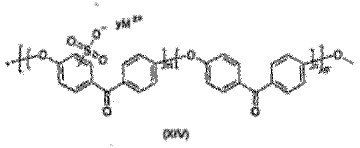 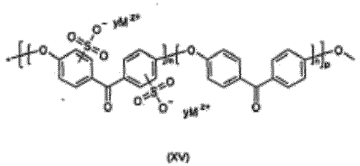 wherein:
- M^{Z+} represents a cation corresponding to an oxidised form of the elements of columns IB, IIB, IIIA, IIIB, IVA, IVB, VA, VB, VIB, VIIB, VIIIB,
- z represents an integer from 1 to 6 corresponding to the degree of oxidation of the metal,
- y represents an integer or fraction equal to 1/z in such a way as to ensure the neutrality between the negative and positive charges,
- m represents the percentage of polymeric units having an oxoaryl or dioxoaryl unit having a sulfonate function. This percentage varies between 0.1 and 100%,
- n represents the percentage of polymeric units having no oxoaryl or dioxoaryl unit functionalised by a sulfonate unit, n varies between 0 and 99.9%,
- p represents the number of polymeric units of the polymer; p varies from 40 to 300.

2. Novel polymers according to claim 1 of formulas I, II, III, IV, V, VI, VII, VIII, IX, X, XI, XII, XIII, XIV and XV wherein:
- M^{Z+} represents a cation corresponding to an oxidised form of titanium, zirconium, vanadium, chromium, molybdenum, tungsten, manganese, iron, copper, silver, gold, zinc, aluminium, gallium, indium, tin, bismuth,
- z represents an integer from 1 to 6 corresponding to the degree of oxidation of the metal,
- y represents an integer or fraction equal to 1/z in such a way as to ensure the neutrality between the negative and positive charges,
- m represents the percentage of polymeric units having an oxoaryl or dioxoaryl unit having a sulfonate function, m varies between 0.1 and 100%,
- n represents the percentage of polymeric units having no oxoaryl or dioxoaryl unit functionalised by a sulfonate unit, n varies between 0 and 99.9%,
- p represents the number of polymeric units of the polymer, p varies from 40 to 300.

3. Novel polymers according to claim 1 of formulas I, II, III, IV, V, VI, VII, VIII, IX, X, XI, XII, XIII, XIV and XV wherein:
- M^{Z+} represents a cation corresponding to an oxidised form of copper, silver, gold, zinc, iron.
- z represents an integer from 1 to 6 corresponding to the degree of oxidation of the metal,
- y represents an integer or fraction equal to 1/z in such a way as to ensure the neutrality between the negative and positive charges,
- m represents the percentage of polymeric units having an oxoaryl or dioxoaryl unit having a sulfonate function, m varies between 0.1 and 100%,
- n represents the percentage of polymeric units having no oxoaryl or dioxoaryl unit functionalised by a sulfonate unit, n varies between 0 and 99.9%,
- p represents the number of polymeric units of the polymer, p varies from 40 to 300.

4. Novel polymers according to claim 1 of formulas I, II, III, IV, V, VI, VII, VIII, IX, X, XI, XII, XIII, XIV and XV wherein:
- M^{Z+} represents a cation corresponding to an oxidised form of copper, silver, gold, zinc, iron.
- z represents an integer from 1 to 6 corresponding to the degree of oxidation of the metal,
- y represents an integer or fraction equal to 1/z in such a way as to ensure the neutrality between the negative and positive charges,
- m represents the percentage of polymeric units having an oxoaryl or dioxoaryl unit having a sulfonate function, m varies between 1 and 100%,
- n represents the percentage of polymeric units having no oxoaryl or dioxoaryl unit functionalised by a sulfonate unit, n varies between 0 and 99%.
- P represents the number of polymeric units of the polymer, p varies from 60 and 200.

5. Method for synthesising polymers according to claims 1 to 4 **characterised in that**
1) the chlorosulfonation is carried out of a polymer of formula XVI, XVII, XVIII, XIX, XX, XXI, XXII, XXIII by a mixture of chlorosulfonic acid, thionyl chloride and of an amide according to an optimised operating procedure, 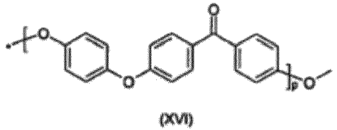 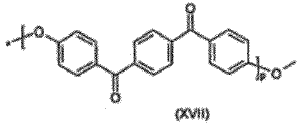 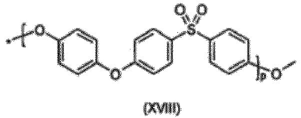 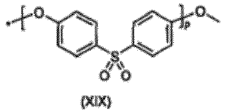 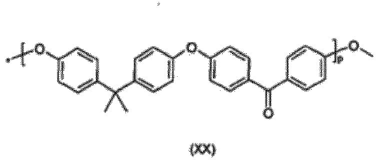 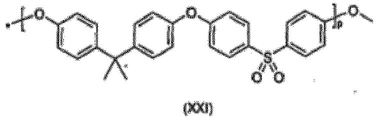 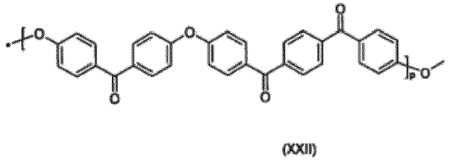 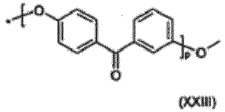 wherein
p represents the number of polymeric units of the polymer, pp varies from 40 to 300,
in order to obtain the polymers of formula XXIV, XXV, XXVI, XVII, XXVIII, XIX, XXX, XXXI, XXXII, XXXIII, XXXIV, XXXV, XXXVI, XXXVII and XXXVIII 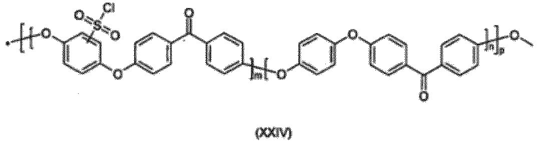 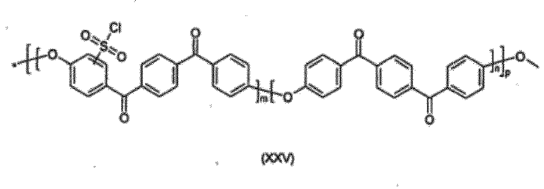 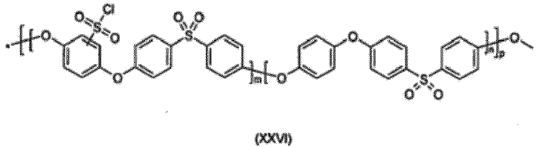 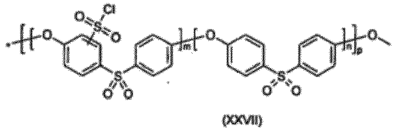 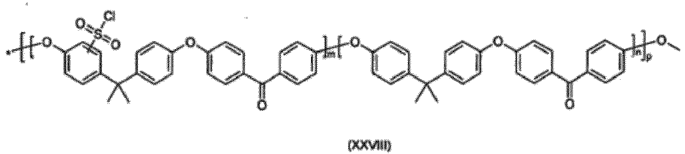 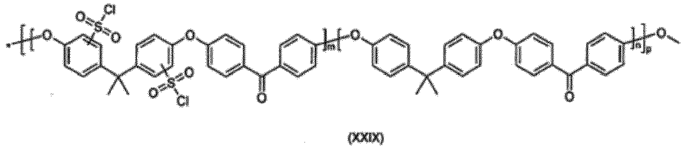 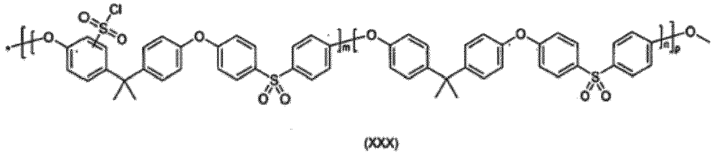 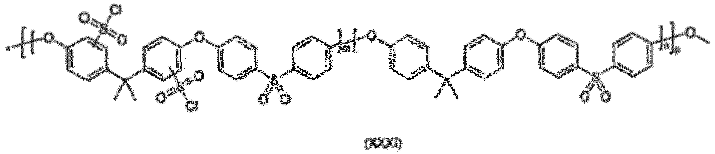 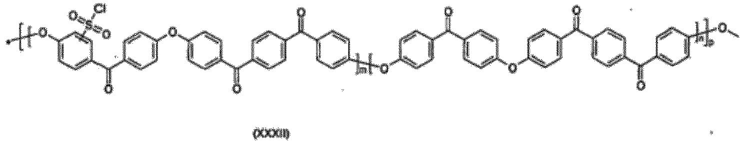 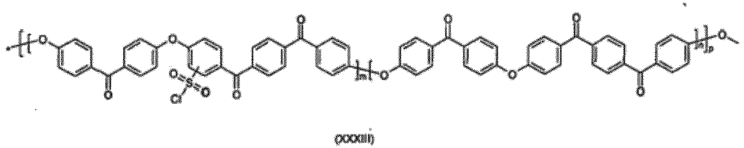 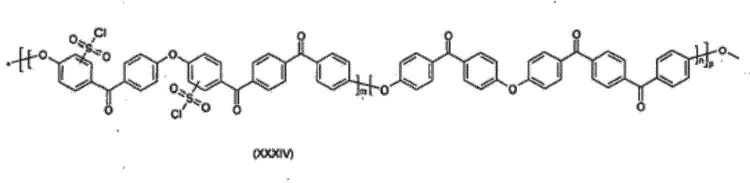 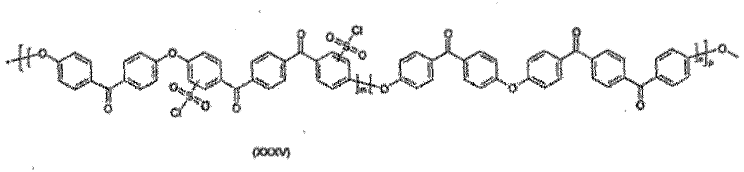 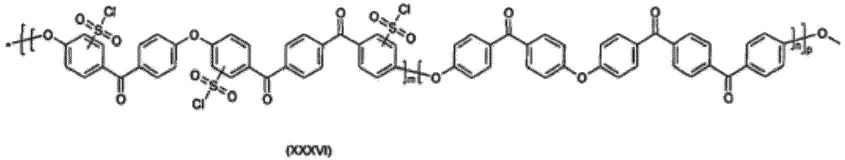 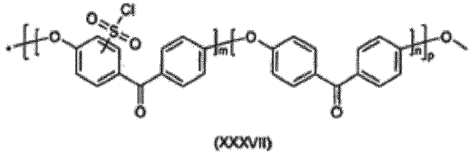 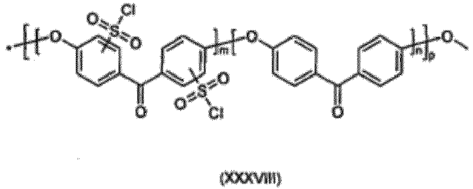 wherein:
- m represents the percentage of polymeric units having an oxoaryl or dioxoaryl unit functionalised by a sulfonyl chloride group, m varies between 0.1 and 100%,
- n represents the percentage of polymeric units having a dioxoaryl unit that is not functionalised by a sulfonyl chloride group, n varies between 0 and 99.9%,
- P represents the number of polymeric units of the polymer, p varies from 40 to 300,
2) then, water is made to react at a temperature between 60 and 140 °C, on the polymers of formula XXIV, XXV, XXVI, XVII, XXVIII, XIX, XXX, XXXI, XXXII, XXXIII, XXXIV, XXXV, XXXVI, XXXVII and XXXVIII in order to obtain the polymers of formula XIL, XL, XLI, XLII, XLIII, XLIV, XLV, XLVI, XLVII, XLVIII, XLIX, L, LI, LII, LIII, 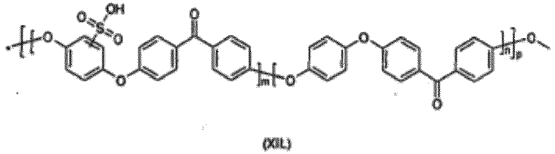 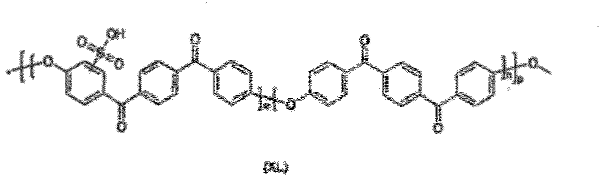 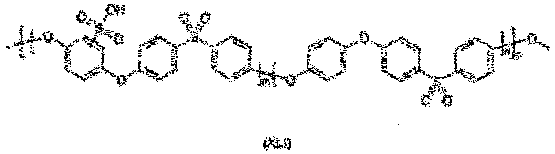 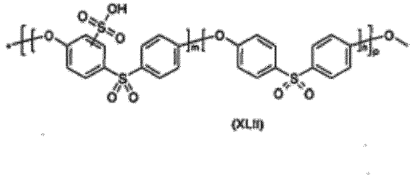 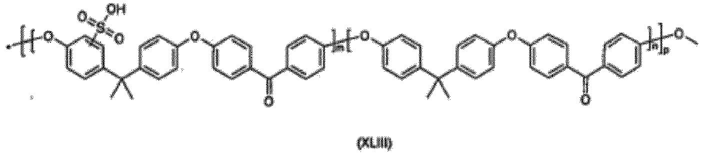 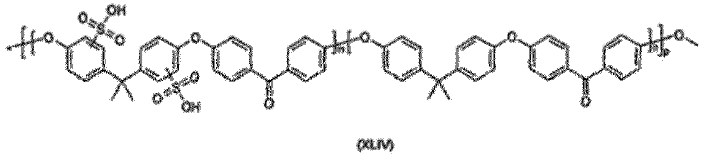 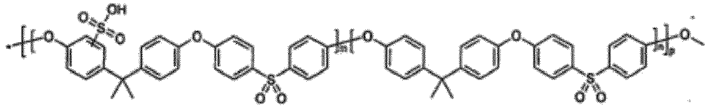 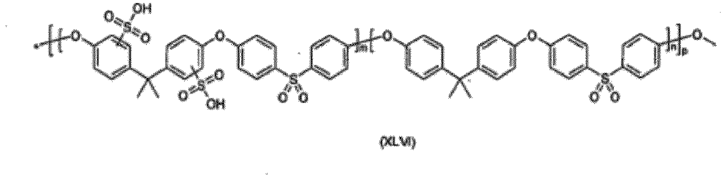 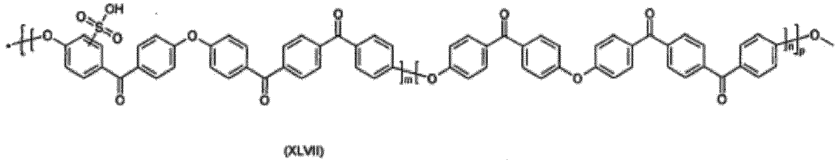 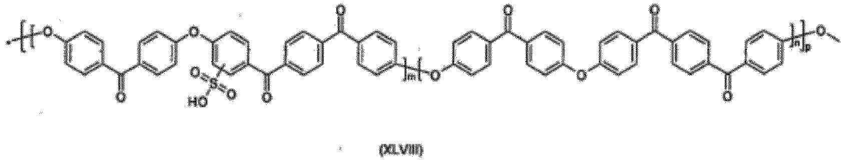 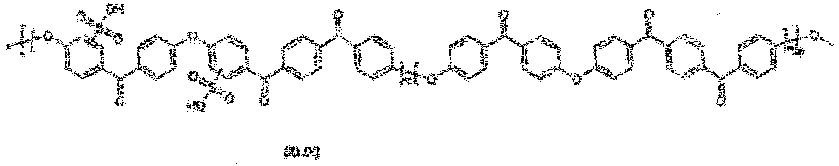 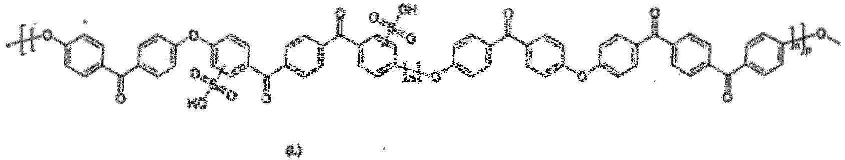 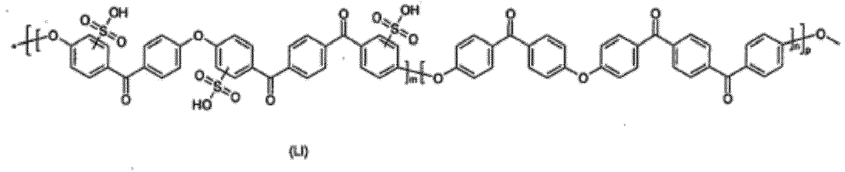 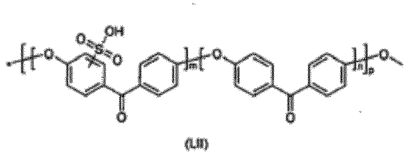 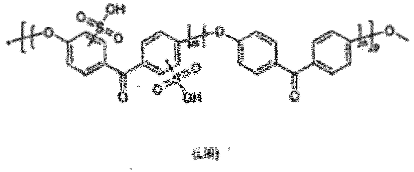 wherein:
- m represents the percentage of polymeric units having an oxoaryl or dioxoaryl unit that is functionalised having a sulfonic function, m varies between 0.1 and 100%,
- n represents the percentage of polymeric units having an oxoaryl or dioxoaryl unit that is not functionalised by a sulfonic function, n varies between 0 and 99.9%,
- P represents the number of polymeric units of the polymer, p varies from 40 to 300,
3) and then, the polymers of formula XIL, XL, XLI, XLII, XLIII, XLIV, XLV, XLVI, XLVII, XLVIII, XLIX, L, LI, LII and LIII are made to react with the metallic salt of the metal M or directly the metal M at the degree of oxidation 0 in the presence of ultrasound.

6. Method for synthesising according to claim 5 **characterised in that**
1) firstly, the chlorosulfonation of a polymer of formula XVI, XVII, XVIII, XIX, XX, XXI, XXII, XXIII by a mixture of chlorosulfonic acid, thionyl chloride and of an amide according to an optimised operating procedure, wherein
p represents the number of polymeric units of the polymer, p varies from 60 and 200,
in order to obtain the polymers of formulas XXIV, XXV, XXVI, XVII, XXVIII, XIX, XXX, XXXI, XXXII, XXXIII, XXXIV, XXXV, XXXVI, XXXVII and XXXVIII wherein:
- m represents the percentage of polymeric units having an oxoaryl or dioxoaryl unit that is functionalised by a chlorosulfonated group, m varies between 1 and 100%.
- n represents the percentage of polymeric units having an oxoaryl or dioxoaryl unit that is not functionalised by a chlorosulfonated group, n varies between 0 and 99%;
- p represents the number of polymeric units of the polymer, p varies from 60 to 200,
2) secondly, water is made to react at a temperature between 90 and 120 °C on the polymers of formula XXIV, XXV, XXVI, XVII, XXVIII, XIX, XXX, XXXI, XXXII, XXXIII, XXXIV, XXXV, XXXVI, XXXVII and XXXVIII in order to obtain the polymers of formulas XIL, XL, XLI, XLII, XLIII, XLIV, XLV, XLVI, XLVII, XLVIII, XLIX, L, LI, LII, LIII, wherein:
- m represents the percentage of polymeric units having an oxoaryl or dioxoaryl unit that is functionalised having a sulfonic function. This percentage varies between 1 and 100%,
- n represents the percentage of polymeric units having an oxoaryl or dioxoaryl unit that is not functionalised by a sulfonic function. This percentage varies between 0 and 99%.
- p represents the number of polymeric units of the polymer; p varies from 60 and 200,
3) and finally, in a first alternative the polymers of formula XIL, XL, XLI, XLII, XLIII, XLIV, XLV, XLVI, XLVII, XLVIII, XLIX, L, LI, LII and LIII are made to react with the metallic salt of the metal M or directly the metal M at the degree of oxidation 0 in the presence of ultrasound.

7. Method according to claims 5 and 6, **characterised in that** the chlorosulfonation is carried out at a temperature between 0 and 80 °C by a mixture of 0.1 to 5 equivalents of chlorosulfonic acid, 0.1 to 15 equivalents of thionyl chloride in the presence of 0.1 to 5 equivalents of an amide in relation to the oxoaryl or dioxoaryl units to be chlorosulfonated in order to obtain rates of chlorosulfonation between 0.1 and 50%.

8. Method according to claim 5 and 6, **characterised in that** the metallic salts used for the reaction with the polymers of formulas XIL, XL, XLI, XLII, XLIII, XLIV, XLV, XLVI, XLVII, XLVIII, XLIX, L, LI, LII and LIII are chlorides, bromides, carbonates, alkyl carboxylates, acetates, propionates, arylcarboxylates, benzoates, acetylacetates, acetylacetonates, alcoolates.

9. Method according to claim 5 and 6, **characterised in that** the preferred metallic salts are acid salts with a low pKa of which the pKa is greater than 0 such as carbonates, acetates, benzoates, alcoolates, acetylacetates, acetylacetonates

10. Method according to claim 5 and 6, **characterised in that** the polymers of formula XIL, XL, XLI, XLII, XLIII, XLIV, XLV, XLVI, XLVII, XLVIII, XLIX, L, LI, LII and LIII are made to react with a metal with a degree of oxidation 0 such as for example indium, iron, copper under ultrasound.

11. The polymers according to claims 1 to 4 of formulas I, II, III, IV, V, VI, VII, VIII, IX, X, XI, XII, XIII, XIV, XV have an antibacterial, fungicide and antimicrobial activity.

12. The polymers according to claims 1 to 4 of formulas I, II, III, IV, V, VI, VII, VIII, IX, X, XI, XII, XIII, XIV, XV have an antibacterial activity.

13. The polymers according to claims 1 to 4 of formulas I, II, III, IV, V, VI, VII, VIII, IX, X, XI, XII, XIII, XIV, XV have an antibacterial activity against for example Staphilococcaus Aureus and Pseudomonas aeruginosa.

14. The polymers according to claims 1 to 4 of formulas I, II, III, IV, V, VI, VII, VIII, IX, X, XI, XII, XIII, XIV, XV have a fungicide activity.

15. The polymers according to claims 1 to 4 of formulas I, II, III, IV, V, VI, VII, VIII, IX, X, XI, XII, XIII, XIV, XV have an antimicrobial activity.

16. The polymers according to claim 5 of formula XIL, XL, XLI, XLII, XLIII, XLIV, XLV, XLVI, XLVII, XLVIII, XLIX, L, LI, LII, LIII have an antimicrobial, fungicide and antibacterial activity.

17. The polymers according to claim 5 of formula XIL, XL, XLI, XLII, XLIII, XLIV, XLV, XLVI, XLVII, XLVIII, XLIX, L, LI, LII, LIII have a fungicide activity.

18. The polymers according to claim 5 of formula XIL, XL, XLI, XLII, XLIII, XLIV, XLV, XLVI, XLVII, XLVIII, XLIX, L, LI, LII, LIII have an antimicrobial activity.

19. The polymers according to claim 5 of formula XIL, XL, XLI, XLII, XLIII, XLIV, XLV, XLVI, XLVII, XLVIII, XLIX, L, LI, LII, LIII have an antibacterial activity.
